(19) Europäisches Patentamt
European Patent Office
Office européen des brevets

(11) **EP 4 763 339 A1**

(12) **EUROPEAN PATENT APPLICATION**
published in accordance with Art. 153(4) EPC

(43) Date of publication:
**24.06.2026   Bulletin 2026/26**

(21) Application number: **24856168.0**

(22) Date of filing: **09.07.2024**

(51) International Patent Classification (IPC):
***B03B 5/00*** *(2006.01)*      ***C12M 1/00*** *(2006.01)*
***C12N 5/00*** *(2006.01)*      ***G01N 15/00*** *(2024.01)*
***G01N 15/01*** *(2024.01)*

(52) Cooperative Patent Classification (CPC):
**B03B 5/00; C12M 1/00; C12N 5/00; G01N 15/00;
G01N 15/01**

(86) International application number:
**PCT/JP2024/024791**

(87) International publication number:
**WO 2025/041473 (27.02.2025 Gazette 2025/09)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **18.08.2023   JP 2023133214**

(71) Applicant: **H.U. Group Holdings, Inc.
Tokyo 107-0052 (JP)**

(72) Inventors:
• **TONOMURA, Wataru
  Akiruno-shi, Tokyo 197-0833 (JP)**
• **KOTAKA, Takeyuki
  Akiruno-shi, Tokyo 197-0833 (JP)**

(74) Representative: **Laine IP Oy
Porkkalankatu 24
00180 Helsinki (FI)**

(54) **SEPARATION DEVICE AND SEPARATION METHOD**

(57)     A separation device and a separation method are provided enabling the usability of separated particles to be guaranteed while improving the efficiency of a separation process.

A separation device 1 includes an inlet port 20, a separation flow path 30 including a first separation flow path 31, a second separation flow path 32, and a third separation flow path 33, and an extraction port 50. The first separation flow path 31 is configured to have a substantially spiral shape, to make a plurality of particles entirely located in an inner wall portion and the like of the first separation flow path 31 as a separation target liquid flows through the first separation flow path 31. The second separation flow path 32 is configured to make the plurality of particles entirely located in an outer wall portion and the like of the third separation flow path 33 as the separation target liquid flows through the second separation flow path 32 and into the third separation flow path 33. The third separation flow path 33 is configured to have a substantially spiral shape, to make the plurality of particles separated according to a plurality of particle sizes as the separation target liquid flows through the third separation flow path 33, and make the plurality of particles located alongside each other in an inner-outer direction in the third separation flow path 33.

Fig. 1

## Description

Technical Field

The present invention relates to a separation device and a separation method.

Background Art

One conventional technique proposed for isolating specific cells from a biological sample involves performing centrifugation on a centrifuge tube containing an insert and a mixture of the biological sample and a separation solution using a centrifuge (see, for example, Patent Document 1). This technique includes a first step of filling the centrifuge tube with the separation solution, a second step of filling the centrifuge tube with the biological sample, a third step of spinning the centrifuge tube in a centrifuge to perform the above-described centrifugation, and finally a fourth step of removing specific cells from the centrifuge tube.

Citation List

Patent Document

Patent Document 1: WO 2012/149641

Summary of the Invention

Technical Problem

The above-described conventional technique includes the first through the fourth steps and thus requires a relatively long time. Furthermore, the second and the fourth steps require complicated operations, which may make it difficult to improve the efficiency of the separation process. Furthermore, if the volume of the above-described mixture exceeds the capacity of the centrifuge tube, the series of the first to the fourth steps needs to be repeated multiple times, which may result in excessive labor for the separation process. Furthermore, spinning the centrifuge tube in the centrifuge in the third step may lead to application of excessive force to specific cells contained in the mixture inside the centrifuge tube, and may result in damaging those specific cells. Thus, the usability of those specific cells after the separation may be difficult to guarantee. Therefore, there is room for improvement in terms of guaranteeing the usability of separated particles, such as specific cells, while improving the efficiency of and reducing the labor required for the separation process.

The present invention is made in view of the above, and an object of the present invention is to provide a separation device and a separation method enabling the usability of separated particles to be guaranteed while improving the efficiency of and reducing the labor required for a separation process.

Means to Solve Problem

In order to solve the above mentioned problem and achieve the purpose, a separation device of claim 1 is a separation device configured to separate a plurality of particles forming a sample contained in a separation target liquid, the separation device comprising: an inlet port through which the separation target liquid is introduced; a separation flow path in which the separation target liquid introduced through the inlet port flows, the separation flow path being configured to separate the plurality of particles of the sample contained in the separation target liquid according to particle sizes; and at least one extraction port configured to extract a particle of a particular particle size among the plurality of particles separated by the separation flow path, wherein the separation flow path includes: a first separation flow path connected with the inlet port; a second separation flow path connected with the first separation flow path; and a third separation flow path connected with the second separation flow path, the first separation flow path is configured to have a substantially arc shape, a substantially spiral shape, or a substantially helical shape, to make the plurality of particles of the sample contained in the separation target liquid entirely located at and/or near an inner wall portion in the first separation flow path as the separation target liquid introduced through the inlet port flows through the first separation flow path, the second separation flow path is configured to make the plurality of particles of the sample contained in the separation target liquid entirely located at and/or near an outer wall portion in the third separation flow path as the separation target liquid flowing out from the first separation flow path flows through the second separation flow path and into the third separation flow path, and the third separation flow path is configured to have a substantially arc shape, a substantially spiral shape, or a substantially helical shape, to make the plurality of particles of the sample contained in the separation target liquid separated according to a plurality of particle sizes as the separation target liquid flowing out from the second separation flow path flows through the third separation flow path, and make the plurality of particles separated located alongside each other in an inner-outer direction from an inner wall portion to the outer wall portion in the third separation flow path.

The separation device of claim 2 according to the separation device of claim 1, wherein the second separation flow path is formed in a substantially S shape.

The separation device of claim 3 according to the separation device of claim 1 or 2, wherein a length of the third separation flow path in the inner-outer direction at a cross section is set to be same as a length of the first separation flow path in the inner-outer direction at a cross section, and a length of the third separation flow path in an orthogonal direction orthogonal to the inner-outer direction at the cross section is set to be longer than a length of the first separation flow path in the orthogonal direction at the cross section.

The separation device of claim 4 according to the separation device of claim 3, wherein the length of the third separation flow path in the orthogonal direction at the cross section is set to increase toward the outer wall portion from the inner wall portion of the third separation flow path.

The separation device of claim 5 according to the separation device of claim 3, wherein the cross section of the third separation flow path is set to have a substantially rectangular shape.

The separation device of claim 6 according to the separation device of claim 1 or 2, wherein the first separation flow path is formed in a substantially spiral shape with multiple turns in a vertical direction, the third separation flow path is formed in a substantially spiral shape with multiple turns in the vertical direction and in a direction opposite to the first separation flow path, the third separation flow path is located more on a downstream side than the first separation flow path, and a downstream end portion of the first separation flow path and an upstream end portion of the third separation flow path are connected via the second separation flow path.

The separation device of claim 7 according to the separation device of claim 6, wherein the first separation flow path has a uniform radius of curvature, and/or the third separation flow path has a uniform radius of curvature.

The separation device of claim 8 according to the separation device of claim 1 or 2 further comprising an extraction flow path connected with the third separation flow path and a plurality of the extraction ports, wherein the extraction flow path includes: a main flow path in which the separation target liquid flowing out from the third separation flow path and containing the plurality of particles separated according to the plurality of particle sizes by the separation flow path flows; and a plurality of branch flow paths in which particles of different particle sizes, among the plurality of particles contained in the separation target liquid flowing out from the main flow path, respectively flow, the branch flow paths branching off from the main flow path, each of the branch flow paths being connected with any of the plurality of extraction ports.

The separation device of claim 9 according to the separation device of claim 1 or 2, wherein the sample is a biological sample containing a plurality of cells.

A separation method of separating of claim 10 is a separation method of separating a plurality of particles forming a sample contained in a separation target liquid using a separation device including an inlet port, a separation flow path connected with the inlet port, and an extraction port connected with the separation flow path, the separation flow path including a first separation flow path that is connected with the inlet port and has a substantially arc shape, a substantially spiral shape, or a substantially helical shape, a second separation flow path connected with the first separation flow path, and a third separation flow path that is connected with the second separation flow path and has a substantially arc shape, a substantially spiral shape, or a substantially helical shape, the separation method comprising: an introduction step of introducing the separation target liquid into the separation flow path through the inlet port; a separation step of separating the plurality of particles of the sample contained in the separation target liquid according to particle sizes as the separation target liquid, introduced in the introduction step, flows through the separation flow path; and an extraction step of extracting a particle of a particular particle size, among the plurality of particles separated in the separation step, through the extraction port, wherein the separation step includes a first separation step of making the plurality of particles of the sample contained in the separation target liquid entirely located at and/or near an inner wall portion in the first separation flow path as the separation target liquid, introduced in the introduction step, flows through the first separation flow path, after the first separation step, a second separation step of making the plurality of particles of the sample contained in the separation target liquid entirely located at and/or near an outer wall portion in the third separation flow path as the separation target liquid flowing out from the first separation flow path flows through the second separation flow path and into the third separation flow path, and after the second separation step, a third separation step of making the plurality of particles of the sample contained in the separation target liquid separated according to the plurality of particle sizes as the separation target liquid flowing out from the second separation flow path flows through the third separation flow path, and making the plurality of particles separated located alongside each other in an inner-outer direction from an inner wall portion to the outer wall portion in the third separation flow path.

Effect of Invention

In the separation device according to claim 1 or the separation method according to claim 10, the first separation flow path is configured to have a substantially arc shape, a substantially spiral shape, or a substantially helical shape, to make the plurality of particles of the sample contained in the separation target liquid entirely located at and/or near an inner wall portion in the first separation flow path as the separation target liquid introduced through the inlet port flows through the first separation flow path, the second separation flow path is configured to make the plurality of particles of the sample contained in the separation target liquid entirely located at and/or near an outer wall portion in the third separation flow path as the separation target liquid flowing out from the first separation flow path flows through the second separation flow path and into the third separation flow path, and the third separation flow path is configured to have a substantially arc shape, a substantially spiral shape, or a substantially helical shape, to make the plurality of particles of the sample contained in the separation target liquid separated according to the plurality of particle sizes as the separation target liquid flowing out from the second separation flow path flows through the third separation flow path, and make the plurality of particles separated located alongside each other in an inner-outer direction from an inner wall portion to the outer wall portion in the third separation flow path. Thus, a simpler and faster separation process than conventional techniques (techniques that separate specific cells using a centrifuge) can be achieved, thereby improving the efficiency of the separation process. Compared with the above-described conventional techniques, even if the amount of the separation target liquid is excessive, an increase in the labor required for the separation process can be suppressed, thereby reducing the labor required for the separation process. Furthermore, compared with the above-described conventional techniques, damage to separated particles that occurs during the separation process can be suppressed, whereby the usability of the separated particles is more likely to be guaranteed.

In the separation device according to claim 2, the second separation flow path is formed in a substantially S shape, so that the plurality of particles of the sample contained in the separation target liquid can be entirely located at and/or near the outer wall portion in the third separation flow path effectively compared with other shapes, whereby the function of the second separation flow path can be improved.

In the separation device according to claim 3, a length of the third separation flow path in the inner-outer direction at a cross section is set to be substantially the same as a length of the first separation flow path in the inner-outer direction at a cross section, and a length of the third separation flow path in an orthogonal direction orthogonal to the inner-outer direction at the cross section is set to be longer than a length of the first separation flow path in the orthogonal direction at the cross section. Thus, the plurality of particles of the sample contained in the separation target liquid can be separated according to the plurality of particle sizes in the third separation flow path effectively compared with other lengths, whereby the separation performance of the third separation flow path can be improved.

In the separation device according to claim 4, the length of the third separation flow path in the orthogonal direction at the cross section is set to increase toward the outer wall portion from the inner wall portion of the third separation flow path. Thus, the plurality of particles of the sample contained in the separation target liquid can be separated according to the plurality of particle sizes in the third separation flow path effectively compared with a case where the third separation flow path has a uniform length in the orthogonal direction at the cross section, whereby the separation performance of the third separation flow path can be further improved.

In the separation device according to claim 5, the cross section of the third separation flow path is set to have a substantially rectangular shape. Thus, the plurality of particles of the sample contained in the separation target liquid can be separated according to the plurality of particle sizes in the third separation flow path effectively compared with a case where the shape is circular or elliptical, whereby the separation performance of the third separation flow path can be further improved.

In the separation device according to claim 6, the first separation flow path is formed in a substantially spiral shape with multiple turns in a vertical direction, the third separation flow path is formed in a substantially spiral shape with multiple turns in the vertical direction and in a direction opposite to that of the first separation flow path, and the third separation flow path is located more on a downstream side than the first separation flow path, and a downstream end portion of the first separation flow path and an upstream end portion of the third separation flow path are connected via the second separation flow path. Thus, compared with a case where the first separation flow path and the third separation flow path are each formed in a helical shape, the first separation flow path and the third separation flow path can function effectively, whereby the separation performance of the separation flow path can be improved. Furthermore, combination and installation of the separation device and other devices (such as, for example, devices that perform pre-processing or detection processing) and/or members (such as, for example, fixing members) can be facilitated, whereby installability of the separation device can be improved.

In the separation device according to claim 7, the first separation flow path has a uniform radius of curvature, and/or the third separation flow path has a uniform radius of curvature. Thus, the distance that the separation target liquid flows through the first separation flow path and/or the third separation flow path can be made long compared with a case where the radius of curvature of the first separation flow path and/or the third separation flow path varies. This allows the first separation flow path and/or the third separation flow path to function effectively (specifically, the plurality of particles contained in the separation target liquid can be stably located at and/or near the inner wall portion in the first separation flow path when the first separation flow path has a uniform radius of curvature, and the above-described plurality of particles can be stably located alongside each other in the inner-outer direction in the third separation flow path when the third separation flow path has a uniform radius of curvature), whereby the separation performance of the separation flow path can be further improved.

In the separation device according to claim 8, the extraction flow path includes: a main flow path; and a plurality of branch flow paths in which particles of different particle sizes, among the plurality of particles contained in the separation target liquid flowing out from the main flow path, respectively flow, the branch flow paths branching off from the main flow path, each of the branch flow paths being connected with any of the plurality of extraction ports. Thus, particles of different particle sizes can be respectively extracted through the plurality of extraction port, whereby the plurality of particles of different particle sizes can be efficiently extracted.

In the separation device according to claim 9, the sample is a biological sample containing a plurality of cells. Thus, a simpler and faster separation process on the plurality of cells can be achieved, thereby further improving the efficiency of the separation process. Furthermore, damage to separated cells that occurs during the separation process can be suppressed, whereby the usability of the separated cells is more likely to be guaranteed.

Brief Description of the Drawings

[FIG. 1] FIG. 1 is a perspective view illustrating an overview of a separation device according to a first embodiment of the present invention.

[FIG. 2] FIG. 2 is a diagram illustrating the separation device in (a) front view and (b) plan view.

[FIG. 3] FIG. 3 is a cross-sectional view of a separation flow path and includes (a) a cross-sectional view of a first separation flow path and (b) a cross-sectional view of a third separation flow path.

[FIG. 4] FIG. 4 is a diagram illustrating a mechanism for separation of a plurality of particles.

[FIG. 5] FIG. 5 is a diagram illustrating a status of separation of the plurality of particles in the separation flow path, and includes (a) a cross-sectional view of an upstream side of the first separation flow path, (b) a cross-sectional view of a downstream side of the first separation flow path, (c) a cross-sectional view of an upstream side of the third separation flow path, and (d) a cross-sectional view of a downstream side of the third separation flow path.

[FIG. 6] FIG. 6 is a perspective view illustrating an overview of a separation device according to a second embodiment.

[FIG. 7] FIG. 7 is a diagram illustrating a separation device according to a modification.

Mode for Carrying Out the Invention

With reference to the attached drawings, embodiments of a separation device and a separation method according to the present invention will be described below in detail. First, [I] the basic concept of the embodiments will be described, then [II] the specific contents of the embodiments will be described, and finally, [III] modification examples of the embodiments will be described. It should be noted that the present invention is not limited by the embodiments.

[I] Basic Concept of Embodiments

First, the basic concept of the embodiments will be described. The embodiments generally relate to a separation device for separating a plurality of particles forming a sample contained in a separation target liquid, and a separation method using this separation device.

The "sample" as used herein refers to a substance that includes a plurality of particles and is subjected to testing, analysis, and/or inspection. This sample conceptually including, for example, specimens, reagents, sludge, and beverages, will be described as a biological sample (specimen) containing a plurality of cells in the embodiments. Examples of the "particle", which could be any type, include cells, bacteria, yeast, viruses, exosomes, microcarriers (for example, latex particles, magnetic particles, gelatin particles), coagulated proteins, Hoppe resin, sand, metal microparticles, and/or the like.

The "specimen" refers to a biological sample that is expected to contain (or is tested to determine whether it contains) a target substance. This specimen conceptually including, for example, clinical specimens (for example, blood such as peripheral blood) and fluids containing physiologically active substances such as low-molecular-weight compounds, will be described as blood in the embodiments.

The "reagent" refers to a substance used in a container described below to detect an analysis target substance through an immune reaction, and conceptually includes, for example, magnetic particle reagents, labeled antibodies, labeled antigens, and the like.

The "sludge" refers to what is discharged as a result of, for example, the treatment for water supply and sewerage systems or for industrial wastewater.

Examples of the "beverage" include soft drinks such as juice and alcoholic beverages such as beer.

The "separation target liquid" refers to a liquid used for sample separation. This separation target liquid conceptually including, for example, a liquid containing only the sample, a liquid containing the sample and a solution (for example, a sheath liquid, a density gradient solution, and/or a diluent), or the like, will be described as a liquid containing only the sample in the embodiments.

[II] Specific Contents of Embodiments

Next, the specific content of the embodiments will be described.

[First Embodiment]

A description will be first given on a separation device and a separation method according to a first embodiment. In the first embodiment, a first separation flow path (described below) and a third separation flow path (described below) each have a substantially spiral shape.

(Configuration)

First of all, a configuration of the separation device according to the first embodiment will be described.

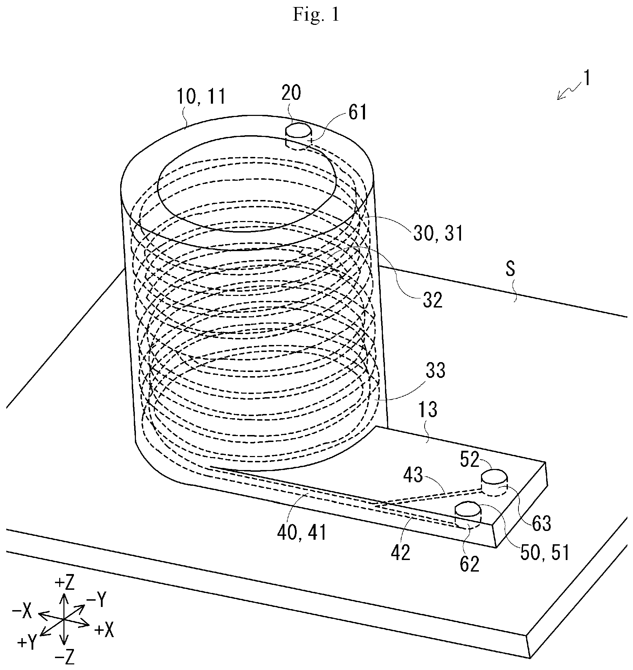

In the following description, the X direction in FIG. 1 will be referred to as the left-right direction of the separation device (-X direction will be the left direction of the separation device, and +X direction will be the right direction of the separation device), the Y direction in FIG. 1 will be referred to as the front-back direction of the separation device (+Y direction will be the front direction of the separation device, and -Y direction will be the rear direction of the separation device), and the Z direction in FIG. 1 will be referred to as the up-down direction of the separation device (+Z direction will be the upward direction of the separation device, and -Z direction will be the downward direction of the separation device).

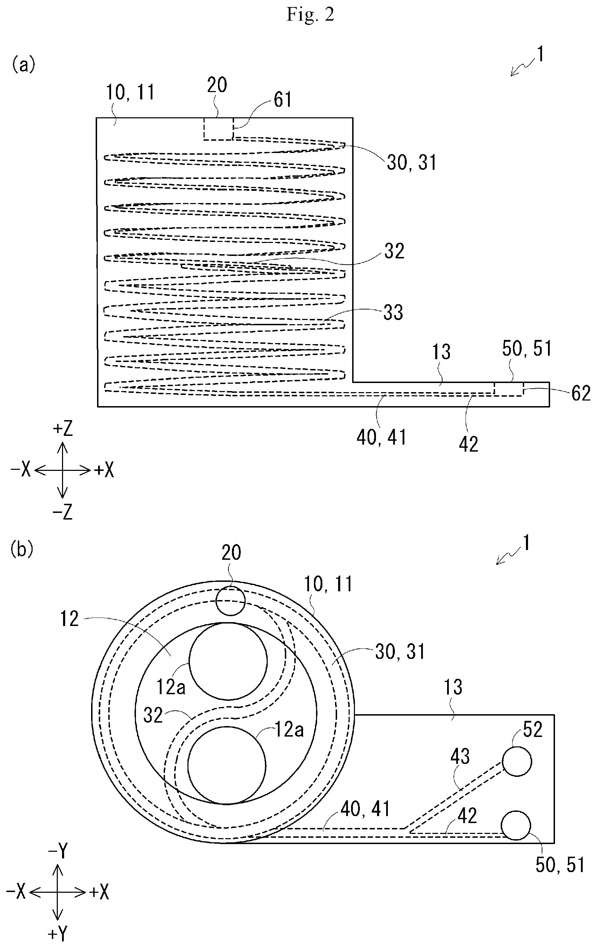

A separation device 1 is a device for separating a plurality of particles P (specifically, a plurality of cells) forming a sample (specifically, a biological sample containing a plurality of cells) contained in a separation target liquid LS, and generally includes a container 10, an inlet port 20, a separation flow path 30, an extraction flow path 40, and an extraction port 50 as illustrated in FIGS. 1 and 2.

(Configuration - Container)

First of all, a configuration of the container 10 will be described.

The container 10 contains the inlet port 20, the separation flow path 30, the extraction port 50, and the extraction flow path 40. As illustrated in FIG. 1, the container 10 is placed on a placement surface S and includes a first containing portion 11, a second containing portion 12, and a third containing portion 13.

(Configuration - Container - First containing portion)

The first containing portion 11 is part of the basic structure of the container 10 and contains the inlet port 20 and part of the separation flow path 30 (specifically, a first separation flow path 31 and a third separation flow path 33 described later). The first containing portion 11 is formed, for example, to have a long, substantially cylindrical body made of resin, and is provided to have the longitudinal direction approximately aligned with the up-down direction as illustrated in FIG. 1.

The shape and the size of the first containing portion 11, which may be arbitrarily set, are specifically set as follows in the first embodiment.

Specifically, the shape of the first containing portion 11 in plan view is set to be substantially annular as illustrated in FIG. 2(b).

However, this should not be construed in a limiting sense, and the shape may be set to be substantially elliptically annular.

Furthermore, as illustrated in FIG. 2(b), the outer diameter of the first containing portion 11 is set to be larger than the outer diameter of the later-described first separation flow path 31 (or the outer diameter of the later-described third separation flow path 33), and, for example, may be set to approximately 50 mm to 80 mm.

Furthermore, as illustrated in FIG. 2(b), the inner diameter of the first containing portion 11 is set to be smaller than the inner diameter of the later-described first separation flow path 31 (or the inner diameter of the later-described third separation flow path 33), and, for example, may be set to approximately 30 mm to 80 mm.

Furthermore, as illustrated in FIG. 2(a), the length of the first containing portion 11 in the up-down direction is set to be longer than the total length of the later-described first separation flow path 31 in the up-down direction and the later-described third separation flow path 33 in the up-down direction, and, for example, may be set to approximately 60 mm to 100 mm.

(Configuration - Container - Second containing portion)

The second containing portion 12 is another part of the basic structure of the container 10, and contains another part of the separation flow path 30 (specifically, a second separation flow path 32 described later). The second containing portion 12 is formed, for example, of a generally plate-shaped body made of resin, and as illustrated in FIG. 2(b), is disposed substantially horizontally within the space surrounded by the inner edges of the first containing portion 11.

Specifically, in the above-described space, the second containing portion 12 is provided at a position corresponding to a downstream end portion (lower end portion) of the later-described first separation flow path 31 and an upstream end portion (upper end portion) of the later-described third separation flow path 33.

The shape and the size of the second containing portion 12, which may be arbitrarily set, are specifically set as follows in the first embodiment.

Specifically, the shape of the second containing portion 12 in plan view is set to be substantially circular as illustrated in FIG. 2(b).

However, this should not be construed in a limiting sense, and the shape may be set to be substantially elliptically annular when the first containing portion 11 has a substantially elliptical shape in plan view, for example. Alternatively, the shape may be set to be substantially rectangular.

The outer diameter of the second containing portion 12 is set to be the same as the inner diameter of the first containing portion 11, as illustrated in FIG. 2(b).

The length of the second containing portion 12 in the up-down direction is set to be longer than the length of the second separation flow path 32 in the up-down direction, as described later, and, for example, may be set to approximately 1 mm to 3 mm.

While the second containing portion 12 may have any specific configuration, in the first embodiment, the second containing portion 12 has one or more (two in FIG. 2(b)) through holes 12a for reducing the weight of the second containing portion 12.

However, this should not be construed in a limiting sense, and three or more through holes 12a may be provided, or the through hole 12a may be omitted, for example.

(Configuration - Container - Third containing portion)

Referring back to FIG. 1, the third containing portion 13 is another part of the basic structure of the container 10 and contains the extraction port 50 and the extraction flow path 40. As illustrated in FIG. 1, the third containing portion 13 is formed, for example, of a plate-shaped body made of resin and is arranged to protrude rightward from the first containing portion 11.

Specifically, the third containing portion 13 is provided at a position corresponding to the downstream end portion (the lower end portion in FIG. 1) of the later-described third separation flow path 33, in the lower portion of the first containing portion 11.

The shape and the size of the third containing portion 13, which may be arbitrarily set, are specifically set as follows in the first embodiment.

Specifically, the shape of the third containing portion 13 in plan view is set to be substantially trapezoidal as illustrated in FIG. 2(b).

However, this should not be construed in a limiting sense, and the shape may be set to be substantially polygonal (such as, for example, substantially rectangular) or substantially elliptical.

Furthermore, the length of the third containing portion 13 in the left-right direction is set to be longer than the length of the extraction flow path 40 in the left-right direction, as illustrated in FIG. 2(b), and, for example, may be set to approximately 50 mm to 70 mm.

The length of the third containing portion 13 in the front-back direction is set to be longer than the length of the extraction flow path 40 in the front-back direction, as illustrated in FIG. 2(b), and, for example, may be set to approximately 20 mm to 50 mm.

The length of the third containing portion 13 in the up-down direction is set to be longer than the length of the extraction flow path 40 in the up-down direction, as illustrated in FIG. 2(a), and, for example, may be set to approximately 2 mm to 5 mm.

(Configuration - Container - Other configurations)

While the other configurations of the container 10 may be arbitrarily set, the first containing portion 11, the second containing portion 12, and the third containing portion 13 are integrally formed in the first embodiment.

Specifically, the portions are formed by molding through 3D printing (or injection molding) using a transparent resin material (such as, for example, polypropylene).

However, this should not be construed in a limiting sense, and the portions may be formed by molding through 3D printing or the like using an opaque resin material.

This configuration allows the container 10 to be constructed easily and quickly, whereby the manufacturability of the container 10 can be improved.

However, this should not be construed in a limiting sense, and, for example, the first containing portion 11, the second containing portion 12, or the third containing portion 13 may be formed separately and then connected using a known connecting unit.

(Configuration - Inlet port)

Referring back to FIG. 1, a configuration of the inlet port 20 will now be described.

The inlet port 20 is an opening for introducing the separation target liquid LS. This inlet port 20 is provided in the first containing portion 11, and specifically, as illustrated in FIG. 1, only one inlet port 20 is provided in the upper surface of the first containing portion 11.

The shape and the size of the inlet port 20, which may be arbitrarily set, are specifically set as follows in the first embodiment.

Specifically, the shape of the inlet port 20 is set to be substantially circular as illustrated in FIG. 1.

However, this should not be construed in a limiting sense, and the shape may be set to be substantially elliptical or substantially polygonal (for example, substantially rectangular), for example.

As illustrated in FIG. 1, the diameter of the inlet port 20 is set to be smaller than the difference between the outer and inner diameters of the first containing portion 11, and, for example, may be set to approximately 5 mm to 10 mm.

(Configuration - Separation flow path)

A configuration of the separation flow path 30 will now be described.

The separation flow path 30 is a flow path through which the separation target liquid LS introduced through the inlet port 20 flows, and is a flow path for separating the plurality of particles P of the sample contained in the separation target liquid LS according to particle sizes. As illustrated in FIGS. 1 and 2, this separation flow path 30 is contained within the container 10 (specifically, the first containing portion 11 and the second containing portion 12), and includes the first separation flow path 31, the second separation flow path 32, and the third separation flow path 33.

(Configuration - Separation flow path - First separation flow path)

The first separation flow path 31 is part of the basic structure of the separation flow path 30 and is a flow path connected with the inlet port 20. This first separation flow path 31 is composed of a long, hollow flow path and is contained within the first containing portion 11.

Specifically, as illustrated in FIG. 2(a), the first separation flow path 31 is located in an upper side portion of the first containing portion 11, and has the upstream end portion (the upper end portion in FIG. 2(a)) connected with the inlet port 20 via a first connection flow path 61.

While the first separation flow path 31 may have any specific configuration, in the first embodiment, the first separation flow path 31 is configured in a substantially arc shape, a substantially spiral shape, or a substantially helical shape, to make the plurality of particles P of the sample contained in the separation target liquid LS entirely located at and/or near an inner wall portion 30a in the first separation flow path 31 as the separation target liquid LS introduced through the inlet port 20 flows through the first separation flow path 31. The specific configuration is as follows.

Here, "arc shape" refers to a two-dimensional or three-dimensional shape that curves like a bow relative to a center point, and is a two-dimensional or three-dimensional arc, elliptical arc, or the like, for example. The "spiral shape" refers to a three-dimensional shape that rotates around an axis over a range of 360 degrees or more and extends at an angle relative to the axis direction, and is, for example, a concept including spirals with a uniform radius of curvature and spirals with a non-uniform radius of curvature. Furthermore, "helical shape" refers to a two-dimensional shape of a helical around a center point.

Furthermore, "the inner wall portion 30a in the first separation flow path 31" refers to the wall portion on the above-described axis side (or the above-described center point side) of the first separation flow path 31 (note that substantially the same applies to the inner wall portion 30a in the third separation flow path 33). Furthermore, "an outer wall portion 30b in the first separation flow path 31" refers to the wall portion on the opposite side of the above-described axis side (or the above-described center point side) of the first separation flow path 31 (note that substantially the same applies to the outer wall portion 30b in the third separation flow path 33).

That is, first, as illustrated in FIG. 2(a), the first separation flow path 31 is formed in a substantially spiral shape with multiple turns in the vertical direction, and more specifically, in a substantially spiral shape with five turns clockwise.

However, this should not be construed in a limiting sense, and the first separation flow path 31 may be formed in a substantially spiral shape with fewer than five turns or six or more turns clockwise, or in a substantially spiral shape with fewer than five turns or five or more turns counterclockwise.

The shape of the first separation flow path 31 in plan view is set to be substantially annular as illustrated in FIG. 2(a).

However, this should not be construed in a limiting sense, and the shape may be set to be substantially elliptically annular.

The radius of curvature of the first separation flow path 31 is set to be uniform as illustrated in FIG. 2(b).

However, this should not be construed in a limiting sense, and the radius of curvature may be set to be non-uniform, and, for example, may be set to decrease (or increase) toward the downward side.

Furthermore, as illustrated in FIG. 2(a), the length of the first separation flow path 31 in the up-down direction is set to be shorter than the length of the first containing portion 11 in the up-down direction, and, for example, is set to be about half the length of the first containing portion 11 in the up-down direction.

However, this should not be construed in a limiting sense, and the length may be set to be longer or shorter than half the length of the first containing portion 11 in the up-down direction.

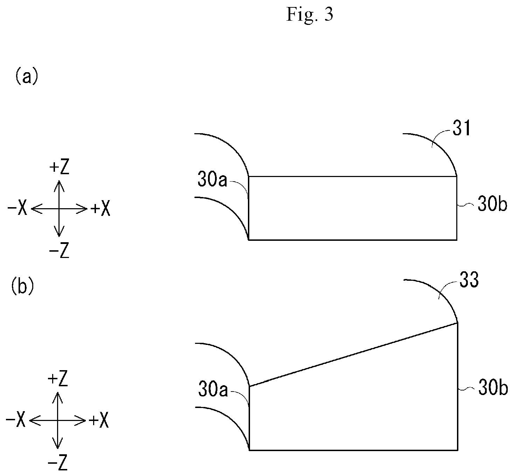

The cross-sectional shape of the first separation flow path 31 is set to be substantially rectangular, as illustrated in FIG. 3(a).

However, this should not be construed in a limiting sense, and, for example, the shape may be set to a shape other than a substantially rectangular shape (such as, for example, a substantially triangular shape, or a substantially elliptical shape).

The cross-sectional size of the first separation flow path 31 is set to such a size that a later-described lift force $F_L$ generated in the first separation flow path 31 acts on the plurality of particles P of the sample contained in the separation target liquid LS in the first separation flow path 31, but a later-described Dean power $F_D$ generated in the first separation flow path 31 is unlikely to act on the plurality of particles P of the sample contained in the separation target liquid LS in the first separation flow path 31.

For example, the length of the cross section of the first separation flow path 31 in the inner-outer direction (the direction from the inner wall portion 30a to the outer wall portion 30b in the separation flow path 30, which is the lateral direction in FIG. 3(a)) may be set to approximately 500 μm to 1000 μm.

Furthermore, the length of the first separation flow path 31 in the direction orthogonal to the inner-outer direction at the cross section (hereinafter referred to as the "orthogonal direction", which is the longitudinal direction in FIG. 3(a)) may be set to be shorter than the length of the first separation flow path 31 in the inner-outer direction at the cross section, and may be set to, for example, approximately 40 μm to 80 μm.

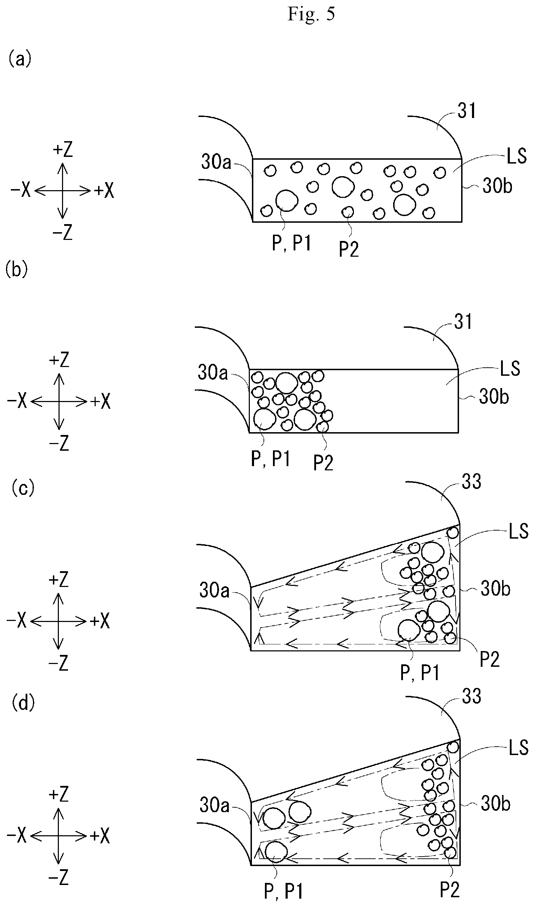

This configuration of the first separation flow path 31 can make, as illustrated in FIG. 5(a) and FIG. 5(b), the plurality of particles P of the sample contained in the separation target liquid LS entirely located at and/or near the inner wall portion 30a in the first separation flow path 31 as the separation target liquid LS introduced through the inlet port 20 flows through the first separation flow path 31, and thus can contribute to the separation of the plurality of particles P.

Furthermore, because the first separation flow path 31 is formed in a substantially spiral shape with multiple turns in the vertical direction, the speed of the separation target liquid LS flowing through the first separation flow path 31 can be made high compared with a case where the first separation flow path 31 is formed in a helical shape. This allows the first separation flow path 31 to function effectively, whereby the separation performance of the separation flow path 30 can be improved.

Furthermore, because the radius of curvature of the first separation flow path 31 is made uniform, the distance that the separation target liquid LS flows through the first separation flow path 31 can be made long compared with a case where the radius of curvature of the first separation flow path 31 varies. This allows the first separation flow path 31 to function effectively (specifically, the plurality of particles P contained in the separation target liquid LS can be stably located at and/or near the inner wall portion 30a in the first separation flow path 31), whereby the separation performance of the separation flow path 30 can be further improved.

(Configuration - Separation flow path - Second separation flow path)

Referring back to FIG. 2, the second separation flow path 32 is another part of the basic structure of the separation flow path 30, and is a flow path connected with the first separation flow path 31. This second separation flow path 32 is composed of a long, hollow flow path and is contained within the second containing portion 12.

Specifically, as illustrated in FIG. 2(b), the second separation flow path 32 is disposed substantially horizontally within the second containing portion 12, and the upstream end portion (specifically, the rear end portion) of the second separation flow path 32 is connected with the downstream end portion (specifically, the lower end portion) of the first separation flow path 31.

While the second separation flow path 32 may have any specific configuration, in the first embodiment, the second separation flow path 32 is configured to make the plurality of particles P of the sample contained in the separation target liquid LS entirely located at and/or near the outer wall portion 30b in the third separation flow path 33 as the separation target liquid LS flowing out from the first separation flow path 31 flows through the second separation flow path 32 and into the third separation flow path 33. The specific configuration is as follows.

Specifically, to begin with, the second separation flow path 32 is formed in a substantially S shape as illustrated in FIG. 2(b) (that is, formed to have a substantially S shape in plan view).

The reason why the second separation flow path 32 is formed in a substantially S shape is as follows.

Specifically, if the second separation flow path 32 is linearly formed, a corner will be formed at the connection portion between the second separation flow path 32 and the first separation flow path 31 or the third flow path, leading to a higher chance of occurrence of turbulence in the flow of the separation target liquid LS. Therefore, the plurality of particles P of the sample contained in the separation target liquid LS at the above-described connecting portion are mixed, leading to the problem of compromising the function of the second separation flow path 32 (the function of locating the plurality of particles P of the sample contained in the separation target liquid LS entirely at and/or near the outer wall portion 30b in the third separation flow path 33). Furthermore, if the second separation flow path 32 is formed in a wavy shape with three or more peaks, the flow of the separation target liquid LS is likely to become turbulent at each peak, leading to the problem of compromising the function of the second separation flow path 32. Therefore, to avoid the above-described problems, the second separation flow path 32 is formed in a substantially S shape.

However, this should not be construed in a limiting sense, and for example, the second separation flow path 32 may be formed in a shape other than a substantially S shape (for example, may be formed linearly or formed in the above-described wavy shape).

The cross-sectional shape of the second separation flow path 32 is set to vary from the upstream side toward the downstream side.

More specifically, the cross-sectional shape of the second separation flow path 32 on the upstream side is set to be substantially the same as or similar to the cross-sectional shape of the first separation flow path 31, and the cross-sectional shape of the second separation flow path 32 on the downstream side is set to be substantially the same as or similar to the cross-sectional shape of the third separation flow path 33.

However, this should not be construed in a limiting sense, and for example, the cross-sectional shape of the second separation flow path 32 may be set to be a uniform shape, and as an example, may be set to a shape that is substantially the same as the cross-sectional shape of the first separation flow path 31 or the cross-sectional shape of the third separation flow path 33.

The cross-sectional size of the second separation flow path 32 is set to such a size that the later-described lift force $F_L$ and the later-described Dean power $F_D$ generated in the second separation flow path 32 are less likely to act on the plurality of particles P of the sample contained in the separation target liquid LS in the second separation flow path 32.

As an example, the length of the second separation flow path 32 in the inner-outer direction at the cross section on the upstream side may be set to be substantially the same as the length of the first separation flow path 31 in the inner-outer direction at the cross section, and the length of the second separation flow path 32 in the orthogonal direction at the cross section on the upstream side may be set to be substantially the same as or slightly longer than the length of the first separation flow path 31 in the orthogonal direction at the cross section.

Furthermore, the length of the second separation flow path 32 in the inner-outer direction at the cross section on the downstream side may be set to be substantially the same as the length of the third separation flow path 33 in the inner-outer direction at the cross section, and the length of the second separation flow path 32 in the orthogonal direction at the cross section on the downstream side may be set to be substantially the same as or slightly shorter than the length of the third separation flow path 33 in the orthogonal direction at the cross section.

This configuration of the second separation flow path 32 can make, as illustrated in FIG. 5(b) and FIG. 5(c), the plurality of particles P of the sample contained in the separation target liquid LS entirely located at and/or near the outer wall portion 30b in the third separation flow path 33 as the separation target liquid LS flowing out from the first separation flow path 31 flows through the second separation flow path 32 and into the third separation flow path 33, and thus can contribute to the separation of the plurality of particles P.

Furthermore, because the second separation flow path 32 is formed in a substantially S shape, the plurality of particles P of the sample contained in the separation target liquid LS can be entirely located at and/or near the outer wall portion 30b in the third separation flow path 33 effectively compared with other shapes, whereby the function of the second separation flow path 32 can be improved.

(Configuration - Separation flow path - Third separation flow path)

Referring back to FIG. 1, the third separation flow path 33 is another part of the basic structure of the separation flow path 30, and is a flow path connected with the second separation flow path 32. This third separation flow path 33 is composed of a long, hollow flow path and is contained within the first containing portion 11.

Specifically, as illustrated in FIG. 1 and FIG. 2(a), the third separation flow path 33 is located in the lower part of the first containing portion 11 (that is, located more on the downstream side than the first separation flow path 31), and has the upstream end portion (the upper end portion in FIG. 2(a)) connected with the downstream end portion of the second separation flow path 32 (the front end portion in FIG. 2(a)) (that is, the downstream end portion of the first separation flow path 31 and the upstream end portion of the third separation flow path 33 are connected via the second separation flow path 32).

While the third separation flow path 33 may have any specific configuration, in the first embodiment, the third separation flow path 33 is configured in a substantially arc shape, a substantially spiral shape, or a substantially helical shape, to make the plurality of particles P of the sample contained in the separation target liquid LS separated according to the plurality of particle sizes as the separation target liquid LS flowing out from the third separation flow path 33 flows through the third separation flow path 33, and make the plurality of particles P separated located alongside each other in the inner-outer direction in the third separation flow path 33. The specific configuration is as follows.

That is, first, as illustrated in FIG. 2(a), the third separation flow path 33 is formed in a substantially spiral shape with multiple turns in the vertical direction and in a direction opposite to that of the first separation flow path 31, and more specifically, in a substantially spiral shape with five turns counterclockwise.

However, this should not be construed in a limiting sense, and the third separation flow path 33 may be formed in a substantially spiral shape with fewer than five turns or six or more turns counterclockwise. Alternatively, if the first separation flow path 31 is formed in a substantially spiral shape with multiple turns counterclockwise, the third separation flow path 33 may be formed in a substantially spiral shape with fewer than five turns or five or more turns clockwise.

The shape of the third separation flow path 33 in plan view is set to be substantially annular.

However, this should not be construed in a limiting sense, and the shape may be set to be substantially elliptically annular.

The radius of curvature of the third separation flow path 33 is set to be uniform.

However, this should not be construed in a limiting sense, and the radius of curvature may be set to be non-uniform, and, for example, may be set to decrease (or increase) toward the downward side.

Furthermore, as illustrated in FIG. 2(a), the length of the third separation flow path 33 in the up-down direction is set to be shorter than the length of the first containing portion 11 in the up-down direction, and, for example, is set to be about half the length of the first containing portion 11 in the up-down direction.

However, this should not be construed in a limiting sense, and the length may be set to be longer or shorter than half the length of the first containing portion 11 in the up-down direction.

The cross-sectional shape of the third separation flow path 33 is set to be substantially trapezoidal, as illustrated in FIG. 3(b).

However, this should not be construed in a limiting sense, and, for example, the shape may be set to a shape other than a substantially trapezoidal shape (such as, for example, a substantially rectangular shape, or a substantially elliptical shape).

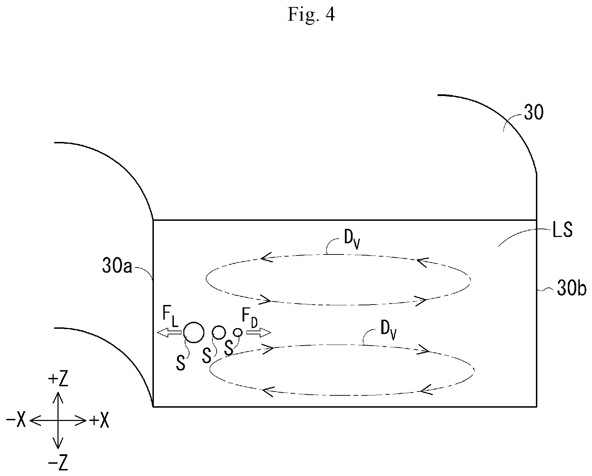

As illustrated in FIG. 4, the cross-sectional size of the third separation flow path 33 is set to such a size that the later-described lift force $F_L$ and the later-described Dean power $F_D$ generated in the third separation flow path 33 act on the plurality of particles P of the sample contained in the separation target liquid LS in the third separation flow path 33, so that the plurality of particles P of the sample contained in the separation target liquid LS are separated according to a plurality of particle sizes.

Specifically, as illustrated in FIG. 5(d), among the above-described plurality of particles P, a particle P2 having a large particle size governed by the lift force $F_L$ is located at or near the inner wall portion 30a in the third separation flow path 33, and a particle P1 having a small particle size governed by the Dean power $F_D$ is located at or near the outer wall portion 30b in the third separation flow path 33.

More specifically, the length of the third separation flow path 33 in the inner-outer direction at the cross section is set to be substantially the same as the length of the first separation flow path 31 in the inner-outer direction at the cross section, as illustrated in FIG. 3(b).

The length of the third separation flow path 33 in the orthogonal direction orthogonal to the inner-outer direction at the cross section is set to be longer than the length of the first separation flow path 31 in the orthogonal direction at the cross section, as illustrated in FIG. 3(b).

In detail, the length of the third separation flow path 33 in the orthogonal direction at the cross section is set to increase from the inner wall portion 30a toward the outer wall portion 30b of the third separation flow path 33.

For example, the minimum length in the above-described orthogonal direction may be set to be substantially the same as (or shorter or longer than) the length of the first separation flow path 31 in the orthogonal direction at the cross section. Furthermore, the maximum length in the above-described orthogonal direction may be set to be approximately 1.5 to 3 times the minimum length in the above-described orthogonal direction.

By thus setting the cross-sectional size of the third separation flow path 33, the center of a later-described Dean vortex $D_V$ is shifted compared with other settings. This promotes the generation of the Dean power $F_D$ within the third separation flow path 33, and the plurality of particles P of the sample contained in the separation target liquid LS can be separated according to the plurality of particle sizes in the third separation flow path 33 effectively. Therefore, the separation performance of the third separation flow path 33 can be improved (an effect similar to the above-described effect should be obtained even if the cross section of the third separation flow path 33 has a substantially rectangular shape).

Furthermore, compared with a case where the length of the third separation flow path 33 in the orthogonal direction at the cross section is made uniform, the plurality of particles P of the sample contained in the separation target liquid LS in the third separation flow path 33 can be separated effectively according to the plurality of particle sizes, whereby the separation performance of the third separation flow path 33 can be further improved.

Here, the Dean power $F_D$ is the force generated by the formation of a secondary flow known as the Dean vortex $D_V$ within the separation flow path 30 as the separation target liquid LS flows through the separation flow path 30 (specifically, the first separation flow path 31 and the third separation flow path 33), as illustrated in FIG. 4, and is calculated based on the following Formulae (1) to (3).

[Math. 1]

$$FD = 5.4 \times 10^{-4}\pi\mu D_e^{1.63}a_p \ ... \ \text{Formula (1)}$$

[Math. 2]

$$D_e = \frac{\rho U_f D_h}{u}\sqrt{\frac{D_h}{2R}} \ ... \ \text{Formula (2)}$$

[Math. 3]

$$D_h = \frac{2ab}{a+b} \ ... \ \text{Formula (3)}$$

(where $\rho$: density (kg/m$^3$),

u: viscosity (kgm$^{-1}$s$^{-1}$),
R: radius of curvature (m),
$D_e$: Dean number
$U_f$: average flow rate (m/s),
$D_h$: hydraulic diameter when the flow path has a rectangular cross section,
a: flow path width, and
b: flow path height)

The lift force $F_L$ is the force generated together with the Dean power $F_D$ within the separation flow path 30 as the separation target liquid LS flows through the separation flow path 30 (specifically, the first separation flow path 31 and the third separation flow path 33), and is calculated based on the following Formula (4).
[Math. 4]

$$F_L = \rho G^2 C_L a_p^4 \ ... \ \text{Formula (4)}$$

(where G: shear rate (m/s),

$C_L$: lift force coefficient, and
$a_p$: particle size (m))

The cross-sectional sizes of the first separation flow path 31 and the third separation flow path 33 are preferably set with reference to Formula (1) to Formula (4).

This configuration of the third separation flow path 33 can make, as illustrated in FIG. 5(c) and FIG. 5(d), the plurality of particles P of the sample contained in the separation target liquid LS separated according to the plurality of particle sizes as the separation target liquid LS flowing out from the second separation flow path 32 flows through the third separation flow path 33, and make the plurality of particles P separated located alongside each other in the inner-outer direction within the third separation flow path 33 (specifically, among the above-described plurality of particles P separated, particles having a large particle size can be located on the inner wall portion 30a side in the third separation flow path 33, and particles having small particle sizes can be located on the outer wall portion 30b side in the third separation flow path 33), and thus can contribute to the separation of the plurality of particles P.

With the third separation flow path 33 formed in a substantially spiral shape with multiple turns in the vertical direction, the third separation flow path 33 can function effectively compared with a case where the third separation flow path 33 is formed in a helical shape, whereby the separation performance of the separation flow path 30 can be improved.

Furthermore, because the radius of curvature of the third separation flow path 33 is made uniform, the distance that the separation target liquid LS flows through the third separation flow path 33 can be made longer compared with a case where the radius of curvature of the third separation flow path 33 varies. This allows the third separation flow path 33 to function effectively (specifically, the plurality of particles P can be located alongside each other stably in the inner-outer direction in the third separation flow path 33), whereby the separation performance of the separation flow path 30 can be further improved.

(Configuration - Separation flow path - Other configurations)

While the other configurations of the separation flow path 30 may be arbitrarily set, the first separation flow path 31, the second separation flow path 32, and the third separation flow path 33 are integrally formed in the first embodiment.

Specifically, the paths are formed integrally by molding through 3D printing (or injection molding) using a transparent resin material, when the container 10 (specifically, the first containing portion 11 and the second containing portion 12) is molded.

This configuration allows the separation flow path 30 to be constructed easily and accurately, whereby the manufacturability of the separation flow path 30 can be improved.

With the separation flow path 30 described above, as the separation target liquid LS introduced through the inlet port 20 flows through the separation flow path 30, the plurality of particles P of the sample contained in the separation target liquid LS can be separated according to particle sizes.

Furthermore, since the substantially spiral first separation flow path 31 and the substantially spiral third separation flow path 33 are connected via the second separation flow path 32, combination and installation of the separation device 1 and other devices (such as, for example, devices that perform pre-processing or detection processing) and/or members (such as, for example, fixing members) can be facilitated, whereby installability of the separation device 1 can be improved.

(Configuration - Extraction flow path)

Referring back to FIG. 1, a configuration of the extraction flow path 40 will now be described.

The extraction flow path 40 is a flow path for extracting the particles P of a specific particle size among the plurality of particles P separated by the separation flow path 30. As illustrated in FIG. 1, the extraction flow path 40 is contained within the third containing portion 13 and includes a main flow path 41, a first branch flow path 42, and a second branch flow path 43.

(Configuration - Extraction flow path - Main flow path)

The main flow path 41 is part of the basic structure of the extraction flow path 40, and is a flow path through which the separation target liquid LS flowing out from the third separation flow path 33 and containing the plurality of particles P separated by the separation flow path 30 according to the plurality of particle sizes flows. This main flow path 41 is composed of a long, hollow flow path, and as illustrated in FIGS. 1 and 2, is installed substantially horizontally within the first containing portion 11 of the third containing portion 13 and is connected with the separation flow path 30 (specifically, the downstream end portion of the third separation flow path 33).

The shape and the size of the main flow path 41, which may be arbitrarily set, are specifically set as follows in the first embodiment.

Specifically, the shape of the main flow path 41 is set to be substantially linear in plan view.

However, this should not be construed in a limiting sense, and the shape may be, for example, set to be substantially curved.

The length of the main flow path 41 in the longitudinal direction (the length in the left-right direction in FIG. 2) is set to be shorter than the length of the third containing portion 13 in the left-right direction. For example, the length may be set to be about half the length of the third containing portion 13 in the left-right direction.

The cross-sectional shape of the main flow path 41 is set to be substantially the same as the cross-sectional shape of the third separation flow path 33.

The cross-sectional size of the main flow path 41 is set to be substantially the same as the cross-sectional size of the third separation flow path 33.

(Configuration - Extraction flow path - First branch flow path and second branch flow path)

The first branch flow path 42 and the second branch flow path 43 are a plurality of branch flow paths in which the particles P of different particle sizes, among the plurality of particles P contained in the separation target liquid LS flowing out from the main flow path 41, respectively flow, the branch flow paths branching off from the main flow path 41, each of the first branch flow path 42 and the second branch flow path 43 being connected with any of a plurality of extraction ports 50.

The first branch flow path 42 and the second branch flow path 43 are long, hollow flow paths that are substantially horizontally installed within the third containing portion 13, as illustrated in FIG. 2, and are connected with the downstream end portion of the main flow path 41.

Specifically, the first branch flow path 42 is arranged substantially along the longitudinal direction of the main flow path 41, and a front wall portion of the first branch flow path 42 is arranged so as to be continuous with the front wall portion of the main flow path 41.

The second branch flow path 43 is arranged at an angle to have an increasing distance from the main flow path 41 rearward toward the downstream side, and a rear wall portion of the second branch flow path 43 is arranged to be continuous with a rear wall portion of the main flow path 41.

The shapes and the sizes of the first branch flow path 42 and the second branch flow path 43, which may be arbitrarily set, are specifically set as follows in the first embodiment.

Specifically, the shapes of the first branch flow path 42 and the second branch flow path 43 are each set to be substantially linear in plan view.

However, this should not be construed in a limiting sense, and the shapes may each be, for example, set to be substantially curved.

The lengths of the first branch flow path 42 and the second branch flow path 43 in the longitudinal direction (the lengths in the left-right direction in FIG. 2) are set to be shorter than the length of the third containing portion 13 in the left-right direction. For example, the lengths may be set to be about half the length of the third containing portion 13 in the left-right direction.

The cross-sectional shapes of the first branch flow path 42 and the second branch flow path 43 are set to be substantially rectangular.

However, this should not be construed in a limiting sense, and for example, shapes other than a substantially rectangular shape (such as, for example, a substantially trapezoidal shape or a substantially elliptical shape) may be set.

The cross-sectional sizes of the first branch flow path 42 and the second branch flow path 43 are set to be smaller than the cross-sectional size of the main flow path 41. For example, the diameters of the cross sections of the first branch flow path 42 and the second branch flow path 43 may be set to be about half the length of the cross section of the main flow path 41 in the horizontal direction (or vertical direction).

With this configuration of the first branch flow path 42, as the particles P1 having small particle sizes, among the plurality of particles P contained in the separation target liquid LS flowing out from the main flow path 41, flow, these small particles P1 can be extracted from the extraction port 50 (specifically, a first extraction port 51 described later) connected with the first branch flow path 42.

Furthermore, with this configuration of the second branch flow path 43, as the particles P2 having large particle sizes, among the plurality of particles P contained in the separation target liquid LS flowing out from the main flow path 41, flow, these large particles P2 can be extracted from the extraction port 50 (specifically, a second extraction port 52 described later) connected with the second branch flow path 43.

(Configuration - Extraction flow path - Other configurations)

While the other configurations of the extraction flow path 40 may be arbitrarily set, the main flow path 41, the first branch flow path 42, and the second branch flow path 43 are integrally formed in the first embodiment.

Specifically, the paths are formed integrally by molding through 3D printing (or injection molding) using a transparent resin material, when the container 10 (specifically, the third containing portion 13) is molded.

This configuration allows the extraction flow path 40 to be constructed easily and accurately, whereby the manufacturability of the extraction flow path 40 can be improved.

With the extraction flow path 40 described above, the particles P of different particle sizes can be extracted through each of the plurality of extraction ports 50, whereby the plurality of particles P of different particle sizes can be efficiently extracted.

(Configuration - Extraction port)

Referring back to FIG. 1, a configuration of the extraction port 50 will be described.

The extraction port 50 is an opening for extracting particles P of a specific particle size among the plurality of particles P separated by the separation flow path 30. As illustrated in FIG. 1, one or more extraction ports 50 are provided in the upper surface of the third containing portion 13, in a portion on the side opposite to the first containing portion 11 side (two ports are provided in FIG. 1).

Specifically, one of the two extraction ports 50 is connected with the downstream end portion of the first branch flow path 42 via a second connection flow path 62. The other of the two extraction ports 50 is connected with the downstream end portion of the second branch flow path 43 via a third connection flow path 63.

In the following, of the two extraction ports 50, the extraction port 51 connected with the first branch flow path 42 will be referred to as the "first extraction port 51," and the extraction port 52 connected with the second branch flow path 43 will be referred to as the "second extraction port 52" as necessary.

The shape and the size of the extraction port 50 (specifically, the first extraction port 51 and the second extraction port 52), which may be arbitrarily set, are specifically set as follows in the first embodiment.

Specifically, the shape of the extraction port 50 is set to be substantially circular.

However, this should not be construed in a limiting sense, and for example, the shape may be set to be substantially elliptical or substantially polygonal (or, for example, a substantially rectangular).

The diameter of the extraction port 50 is set to be substantially the same as the diameter of the inlet port 20, and for example, may be set to approximately 5 mm to 10 mm.

However, this should not be construed in a limiting sense, and for example, the diameter may be set to be shorter or longer than the diameter of the inlet port 20.

(Configuration - Other configurations)

While the other configurations of the separation device 1 may be arbitrarily set, the container 10, the inlet port 20, the separation flow path 30, the extraction flow path 40, and the extraction port 50 are integrally formed in the first embodiment.

Specifically, the members are formed integrally by molding through 3D printing (or injection molding) using a transparent resin material, when the container 10 is molded.

This configuration allows the separation device 1 to be constructed easily and accurately, whereby the manufacturability of the separation device 1 can be improved.

Furthermore, with the separation device 1 described above, a simpler and faster separation process than conventional techniques (techniques that separate specific cells using a centrifuge) can be achieved, thereby improving the efficiency of the separation process. Furthermore, compared with the above-described conventional techniques, damage to the separated particles P that occurs during the separation process can be suppressed, whereby the usability of the separated particles P can be more reliably guaranteed.

Furthermore, since the sample is a biological sample containing a plurality of cells, a simpler and faster separation process for the plurality of cells can be achieved, further improving the efficiency of the separation process. In addition, damage to the separated cells that occurs during the above-described separation process can be suppressed, whereby the usability of the separated cells can be more reliably guaranteed.

(Separation method)

Next, a separation method using the separation device 1 described above will be described.

The separation method is a method for separating the plurality of particles P forming the sample contained in the separation target liquid LS using the separation device 1. This separation method includes an introduction step, a separation step, and an extraction step.

(Separation method - Introduction step)

First of all, the introduction step will be described.

The introduction step is a step of introducing the separation target liquid LS into the separation flow path 30 through the inlet port 20.

Specifically, the separation target liquid LS is introduced through injection into the inlet port 20 using a known injection unit (such as, for example, a pipette, not illustrated) inserted into the inlet port 20.

(Separation method - Separation step)

Next, the separation step will be described.

The separation step is a step, after the introduction step, of separating the plurality of particles P of the sample contained in the separation target liquid LS according to particle sizes as the separation target liquid LS, introduced in the introduction step, flows through the separation flow path 30, and includes a first separation step, a second separation step, and a third separation step.

(Separation method - Separation step - First separation step)

First of all, the first separation step will be described.

The first separation step is a step of making the plurality of particles P of the sample contained in the separation target liquid LS entirely located at and/or near the inner wall portion 30a in the first separation flow path 31 as the separation target liquid LS, introduced in the introduction step, flows through the first separation flow path 31.

Specifically, as illustrated in FIG. 5(a), in the upstream portion of the first separation flow path 31, the plurality of particles P of the sample contained in the separation target liquid LS are dispersed. Then, the lift force $F_L$ generated in the separation target liquid LS flowing through the substantially spiral first separation flow path 31 acts on the plurality of particles P, making the plurality of particles P collected at and/or near the inner wall portion 30a in the first separation flow path 31 in the downstream portion of the first separation flow path 31 as illustrated in FIG. 5(b). As a result, the plurality of particles P are entirely located at and/or near the inner wall portion 30a in the first separation flow path 31.

(Separation method - Separation step - Second separation step)

Next, the second separation step will be described.
The second separation step is a step, after the first separation step, of making the plurality of particles P of the sample contained in the separation target liquid LS entirely located at and/or near the outer wall portion 30b in the third separation flow path 33 as the separation target liquid LS flowing out from the first separation flow path 31 flows through the second separation flow path 32 and into the third separation flow path 33.
Specifically, as illustrated in FIG. 5(b), in the downstream side portion of the first separation flow path 31, the plurality of particles P of the sample contained in the separation target liquid LS are entirely located at and/or near the inner wall portion 30a in the first separation flow path 31. Then, the above-described separation target liquid LS flows through the second separation flow path 32 and into the third separation flow path 33, which is substantially spiral and wound in the direction opposite to that of the first separation flow path 31. As a result, as illustrated in FIG. 5(c), in the upstream portion of the third separation flow path 33, the plurality of particles P are entirely located at and/or near the outer wall portion 30b in the third separation flow path 33.

(Separation method - Separation step - Third separation step)

Next, the third separation step will be described.
The third separation step is a step, after the second separation step, of making the plurality of particles P of the sample contained in the separation target liquid LS separated according to the plurality of particle sizes as the separation target liquid LS flowing out from the second separation flow path 32 flows through the third separation flow path 33, and making the plurality of particles P separated located alongside each other in the inner-outer direction in the third separation flow path 33.
Specifically, as illustrated in FIG. 5(c), in the upstream portion of the third separation flow path 33, the plurality of particles P of the sample contained in the separation target liquid LS are located at and/or near the outer wall portion 30b in the third separation flow path 33. Subsequently, the lift force $F_L$ and the Dean force $F_D$ generated in the separation target liquid LS flowing in the substantially spiral third separation flow path 33 act on the plurality of particles P, making the particles P2 having large particle sizes, among the plurality of particles P, collected at or near the inner wall portion 30a in the third separation flow path 33 in the downstream side of the third separation flow path 33, and making the particles P1 having small particle sizes collected at or near the outer wall portion 30b in the third separation flow path 33, thereby locating the large particles P2 and the small particles P1 alongside each other in the inner-outer direction as illustrated in FIG. 5(d).

(Separation method - Extraction step)

Next, the extraction step will be described.
The extraction step is a step, after the separation step, of extracting particles P of a specific particle size, among the plurality of particles P separated in the separation step, through the extraction port 50.
Specifically, as the particles P1 having small particle sizes, among the plurality of particles P separated in the separation step, flow into the first branch flow path 42 through the main flow path 41 of the extraction flow path 40, the small particles P1 are sucked from the first extraction port 51 using a known suction unit (such as, for example, a pipette, not illustrated) inserted into the first extraction port 51 to extract the small particles P1.
Furthermore, as the particles P2 having large particle sizes, among the plurality of particles P separated in the separation step, flow into the second branch flow path 43 through the main flow path 41 of the extraction flow path 40, the large particles P2 are sucked from the second extraction port 52 using the above-described suction unit inserted into the second extraction port 52 to extract the large particles P2.
With the separation method described above, a simpler and faster separation process than conventional techniques (techniques that separate specific cells using a centrifuge) can be achieved, thereby improving the efficiency of the separation process. Furthermore, compared with the above-described conventional techniques, damage to separated particles P that occurs during the separation process can be suppressed, whereby the usability of the separated particles P is more likely to be guaranteed.

(Effects of First Embodiment)

According to the first embodiment, the first separation flow path 31 is configured to have a substantially spiral shape to make the plurality of particles P of the sample contained in the separation target liquid LS entirely located at and/or near an inner wall portion 30a in the first separation flow path 31 as the separation target liquid LS introduced through the inlet port 20 flows through the first separation flow path 31, the second separation flow path 32 is configured to make the plurality of particles P of the sample contained in the separation target liquid LS entirely located at and/or near an outer wall portion 30b in the third separation flow path 33 as the separation target liquid LS flowing out from the first separation flow path 31 flows through the second separation flow path 32 and into the third separation flow path 33, and the third separation flow path 33 is configured to have a substantially spiral shape to make the plurality of particles P of the sample contained in the separation target liquid LS separated according to the plurality of particle P sizes as the separation target liquid LS flowing out from the second separation flow path 32 flows through the third separation flow path 33, and make the plurality of particles P separated located alongside each other in an inner-outer direction from an inner wall portion 30a to the outer wall portion 30b in the third separation flow path 33. Thus, a simpler and faster separation process than conventional techniques (techniques that separate specific cells using a centrifuge) can be achieved, thereby improving the efficiency of the separation process. Compared with the above-described conventional techniques, even if the amount of the separation target liquid LS is excessive, an increase in the labor required for the separation process can be suppressed, thereby reducing the labor required for the separation process. Furthermore, compared with the above-described conventional techniques, damage to separated particles P that occurs during the separation process can be suppressed, whereby the usability of the separated particles P is more likely to be guaranteed.

In addition, the second separation flow path 32 is formed in a substantially S shape, so that the plurality of particles P of the sample contained in the separation target liquid LS can be entirely located at and/or near the outer wall portion 30b in the third separation flow path 33 effectively compared with other shapes, whereby the function of the second separation flow path 32 can be improved.

In addition, a length of the third separation flow path 33 in the inner-outer direction at a cross section is set to be substantially the same as a length of the first separation flow path 31 in the inner-outer direction at a cross section, and a length of the third separation flow path 33 in an orthogonal direction orthogonal to the inner-outer direction at the cross section is set to be longer than a length of the first separation flow path 31 in the orthogonal direction at the cross section. Thus, the plurality of particles P of the sample contained in the separation target liquid LS can be separated according to the plurality of particle sizes in the third separation flow path 33 effectively comparted with other lengths, whereby the separation performance of the third separation flow path 33 can be improved.

In addition, the length of the third separation flow path 33 in the orthogonal direction at the cross section is set to increase toward the outer wall portion 30b from the inner wall portion 30a of the third separation flow path 33. Thus, the plurality of particles P of the sample contained in the separation target liquid LS can be separated according to the plurality of particle sizes in the third separation flow path 33 effectively compared with a case where the third separation flow path 33 has a uniform length in the orthogonal direction at the cross section, whereby the separation performance of the third separation flow path 33 can be further improved.

In addition, the first separation flow path 31 is formed in a substantially spiral shape with multiple turns in a vertical direction, the third separation flow path 33 is formed in a substantially spiral shape with multiple turns in the vertical direction and in a direction opposite to that of the first separation flow path 31, and the third separation flow path 33 is located more on a downstream side than the first separation flow path 31, and a downstream end portion of the first separation flow path 31 and an upstream end portion of the third separation flow path 33 are connected via the second separation flow path 32. Thus, compared with a case where the first separation flow path 31 and the third separation flow path 33 are each formed in a helical shape, the first separation flow path 31 and the third separation flow path 33 can function effectively, whereby the separation performance of the separation flow path can be improved. Furthermore, combination and installation of the separation device 1 and other devices (such as, for example, devices that perform pre-processing or detection processing) and/or members (such as, for example, fixing members) can be facilitated, whereby installability of the separation device 1 can be improved.

In addition, the first separation flow path 31 has a uniform radius of curvature, and the third separation flow path 33 has a uniform radius of curvature. Thus, the distance that the separation target liquid LS flows through the first separation flow path 31 and the third separation flow path can be made long compared with a case where the radius of curvature of the first separation flow path 31 and the third separation flow path 33 varies. This allows the first separation flow path 31 and the third separation flow path to function effectively (specifically, the plurality of particles P contained in the separation target liquid LS can be stably located at and/or near the inner wall portion 30a in the first separation flow path 31 when the first separation flow path 31 has a uniform radius of curvature, and the above-described plurality of particles P can be stably located alongside each other in the inner-outer direction in the third separation flow path 33 when the third separation flow path 33 has a uniform radius of curvature), whereby the separation performance of the separation flow path can be further improved.

In addition, the extraction flow path 40 includes: a main flow path 41; and a plurality of branch flow paths in which particles P of different particle sizes, among the plurality of particles P contained in the separation target liquid LS flowing out from the main flow path 41, respectively flow, the branch flow paths branching off from the main flow path 41, each of the branch flow paths being connected with any of the plurality of extraction ports 50. Thus, particles P of different particle sizes can be respectively extracted through the plurality of extraction port 50, whereby the plurality of particles P of different particle sizes can be efficiently extracted.

In addition, the sample is a biological sample containing a plurality of cells. Thus, a simpler and faster separation process on the plurality of cells can be achieved, thereby further improving the efficiency of the separation process. Furthermore, damage to separated cells that occurs during the separation process can be suppressed, whereby the usability of the separated cells is more likely to be guaranteed.

[Second Embodiment]

Next, a separation device according to a second embodiment will be described. In the second embodiment, the first separation flow path and the third separation flow path are each substantially helical. The configuration according to the second embodiment is the same as the configuration according to the first embodiment unless otherwise specified. For the same configuration, the same names or symbols as those used in the first embodiment will be used as necessary, and the description thereof will be omitted.

(Configuration)

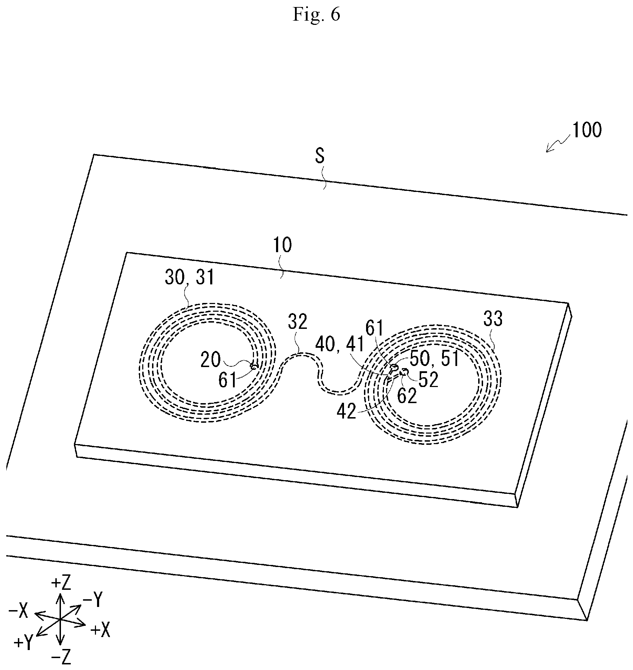

First of all, a configuration of the separation device according to the second embodiment will be described. A separation device 100 according to the second embodiment generally includes, as illustrated in FIG. 6, the container 10, the inlet port 20, the separation flow path 30, the extraction flow path 40, and the extraction port 50.

(Configuration - Container)

To begin with, a configuration of the container 10 will be described.
The container 10 is formed, for example, of a solid body made of resin (specifically, as illustrated in FIG. 6, it is formed of a substantially flat plate-shaped body), and is placed on the substantially horizontal placement surface S, as illustrated in FIG. 6.
The shape and the size of the container 10, which may be arbitrarily set, are specifically set as follows in the second embodiment.
Specifically, the shape of the container 10 in plan view is set to be substantially rectangular as illustrated in FIG. 6. However, this should not be construed in a limiting sense, and, for example, the shape may be set to be substantially circular or substantially elliptically annular.
As illustrated in FIG. 6, the length of the container 10 in the left-right direction is set to be longer than the length of the separation flow path 30 in the left-right direction, and, for example, may be set to be approximately 1.2 to 2 times the length of the separation flow path 30 in the left-right direction.
As illustrated in FIG. 6, the length of the container 10 in the front-back direction is set to be longer than the length of the separation flow path 30 in the front-back direction, and for example, may be set to be approximately 1.2 to 2 times the length of the separation flow path 30 in the front-back direction.
The length of the container 10 in the up-down direction is set to be longer than the length of the separation flow path 30 in the up-down direction, and for example, may be set to approximately two to three times the length of the separation flow path 30 in the front-back direction.

(Configuration - Inlet port)

Next, a configuration of the inlet port 20 will be described.
The inlet port 20 has substantially the same configuration as the inlet port 20 of the first embodiment, and as illustrated in FIG. 6, only one inlet port 20 is provided in the upper surface of the container 10, specifically, in a left side portion of the upper surface of the container 10.

(Configuration - Separation flow path)

Next, a configuration of the separation flow path 30 will be described.
As illustrated in FIG. 6, the separation flow path 30 is contained within the container 10 and includes the first separation flow path 31, the second separation flow path 32, and the third separation flow path 33.

(Configuration - Separation flow path - First separation flow path)

The first separation flow path 31 is composed of a long, hollow flow path, and as illustrated in FIG. 6, is installed substantially horizontally on the left side of the container 10. The upstream end portion of the first separation flow path 31 (the inner end portion in FIG. 6) is connected with the inlet port 20 via the first connection flow path 61.
While the first separation flow path 31 may have any specific configuration, in the second embodiment, the first separation flow path 31 is configured to have a substantially arc shape, a substantially spiral shape, or a substantially helical shape, to make the plurality of particles P of the sample contained in the separation target liquid LS entirely located at and/or near the inner wall portion 30a in the first separation flow path 31 as the separation target liquid LS introduced through the inlet port 20 flows through the first separation flow path 31. The specific configuration is as follows.
First, as illustrated in FIG. 6, the first separation flow path 31 is formed in a substantially helical shape with multiple turns in plan view, and more specifically, formed in a substantially helical shape with three turns counterclockwise from the inside to the outside of the container 10.
However, this should not be construed in a limiting sense, and the first separation flow path 31 may be formed in a substantially helical shape with fewer than three turns or four or more turns counterclockwise, or in a substantially helical shape with fewer than three turns or three or more turns clockwise.

As illustrated in FIG. 6, the length of the first separation flow path 31 in the left-right direction is set to be shorter than the length of the container 10 in the left-right direction, and for example, is set to be about one-third of the length of the container 10 in the left-right direction.

As illustrated in FIG. 6, the length of the first separation flow path 31 in the front-back direction is set to be shorter than the length of the container 10 in the front-back direction, and for example, is set to about half the length of the container 10 in the front-back direction.

The cross-sectional shape of the first separation flow path 31 is set to be substantially the same as the cross-sectional shape of the first separation flow path 31 according to the first embodiment.

The cross-sectional size of the first separation flow path 31 is set to be substantially the same as the cross-sectional size of the first separation flow path 31 according to the first embodiment.

This configuration of the first separation flow path 31 can make the plurality of particles P of the sample contained in the separation target liquid LS entirely located at and/or near the inner wall portion 30a in the first separation flow path 31 as the separation target liquid LS introduced through the inlet port 20 flows through the first separation flow path 31, and thus can contribute to the separation of the plurality of particles P, as with the first separation flow path 31 according to the first embodiment.

(Configuration - Separation flow path - Second separation flow path)

The second separation flow path 32 is composed of a long, hollow flow path and is contained within the container 10.

Specifically, the second separation flow path 32 is substantially horizontally arranged in a center portion of the container 10, and the upstream end portion of the second separation flow path 32 (the left end portion in FIG. 6) is connected with the downstream end portion of the first separation flow path 31 (the outer end portion in FIG. 6).

While the second separation flow path 32 may have any specific configuration, in the second embodiment, the second separation flow path 32 is configured to make the plurality of particles P of the sample contained in the separation target liquid LS entirely located at and/or near the outer wall portion 30b in the third separation flow path 33 as the separation target liquid LS flowing out from the first separation flow path 31 flows through the second separation flow path 32 and into the third separation flow path 33. The specific configuration is as follows.

Specifically, to begin with, as illustrated in FIG.

6, the second separation flow path 32 is formed in a substantially S shape (specifically, formed in a substantially S shape in plan view).

The cross-sectional shape of the second separation flow path 32 is set to be substantially the same as the cross-sectional shape of the second separation flow path 32 according to the first embodiment.

The cross-sectional size of the second separation flow path 32 is set to be substantially the same as the cross-sectional size of the second separation flow path 32 according to the first embodiment.

This configuration of the second separation flow path 32 can make the plurality of particles P of the sample contained in the separation target liquid LS entirely located at and/or near the outer wall portion 30b in the third separation flow path 33 as the separation target liquid LS flowing out from the first separation flow path 31 flows through the second separation flow path 32 and into the third separation flow path 33, and thus can contribute to the separation of the plurality of particles P, as with the second separation flow path 32 according to the first embodiment.

(Configuration - Separation flow path - Third separation flow path)

The third separation flow path 33 is composed of a long, hollow flow path and is contained in the container 10.

Specifically, as illustrated in FIG. 6, the third separation flow path 33 is placed on the right side of the container 10 (specifically, arranged substantially horizontally alongside the first separation flow path 31), and the upstream end portion of the third separation flow path 33 (the outer end portion in FIG. 6) is connected with the downstream end portion of the second separation flow path 32 (the right end portion in FIG. 6).

While the third separation flow path 33 may have any specific configuration, in the second embodiment, the third separation flow path 33 is configured to have a substantially arc shape, a substantially spiral shape, or a substantially helical shape, to make the plurality of particles P of the sample contained in the separation target liquid LS separated in accordance with a plurality of particle sizes as the separation target liquid LS flowing out from the third separation flow path 33 flows through the third separation flow path 33, and make the plurality of particles P separated located alongside each other in the inner-outer direction in the third separation flow path 33. The specific configuration is as follows.

Specifically, first, as illustrated in FIG. 6, the third separation flow path 33 is formed in a substantially helical shape with multiple turns in plan view and in the direction opposite to that of the first separation flow path 31, and more specifically, is formed in a substantially helical shape with three turns clockwise from the outside to the inside of the container 10.

However, this should not be construed in a limiting sense, and the third separation flow path 33 may be formed in a substantially helical shape with fewer than three turns or four or more turns clockwise. Alternatively, if the first separation flow path 31 is formed in a substantially helical shape with multiple turns clockwise, the third separation flow path 33 may be formed in a substantially helical shape with fewer than three turns or three or more turns counterclockwise.

As illustrated in FIG. 6, the length of the third separation flow path 33 in the left-right direction is set to be shorter than the length of the container 10 in the left-right direction, and for example, is set to about one-third of the length of the container 10 in the left-right direction.

As illustrated in FIG. 6, the length of the third separation flow path 33 in the front-back direction is set to be shorter than the length of the container 10 in the front-back direction, and for example, is set to be about half the length of the container 10 in the front-back direction.

The cross-sectional shape of the third separation flow path 33 is set to be substantially the same as the cross-sectional shape of the third separation flow path 33 according to the first embodiment.

The cross-sectional size of the third separation flow path 33 is set to be substantially the same as the cross-sectional size of the third separation flow path 33 according to the first embodiment.

With the cross-sectional size of the third separation flow path 33 thus set, the plurality of particles P of the sample contained in the separation target liquid LS can be separated according to a plurality of particle sizes effectively in the third separation flow path 33, and the separation performance of the third separation flow path 33 can be improved, as with the third separation flow path 33 according to the first embodiment.

(Configuration - Extraction flow path)

Next, a configuration of the extraction flow path 40 will be described.

As illustrated in FIG. 6, the extraction flow path 40 is contained in the container 10 and includes the first branch flow path 42 and the second branch flow path 43.

(Configuration - Extraction flow path - First branch flow path and second branch flow path)

The first branch flow path 42 and the second branch flow path 43 are a plurality of branch flow paths branching off from the separation flow path 30 (specifically, the third separation flow path 33), and each of the first branch flow path 42 and the second branch flow path 43 is connected with any of the plurality of extraction ports 50, and particles P of different particle sizes, among the plurality of particles P contained in the separation target liquid LS flowing out from the main flow path 41, flow in each of the first branch flow path 42 and the second branch flow path 43.

The first branch flow path 42 and the second branch flow path 43 are formed as long, hollow flow paths, and as illustrated in FIG. 6, are each installed substantially horizontally within the container 10 and connected with the downstream end portion of the third separation flow path 33.

Specifically, the first branch flow path 42 is arranged substantially along the longitudinal direction of the third separation flow path 33, and the outer wall portion of the first branch flow path 42 is arranged to be continuous with the outer wall portion 30b of the third separation flow path 33.

The second branch flow path 43 is arranged to have an increasing distance from the third separation flow path 33 inward of the container 10 toward the downstream side, and the inner wall portion of the second branch flow path 43 is arranged to be continuous with the inner wall portion 30a of the third separation flow path 33.

The specific shapes and sizes of the first branch flow path 42 and the second branch flow path 43 are set to be substantially the same as the specific shapes and sizes of the first branch flow path 42 and the second branch flow path 43 according to the first embodiment.

With this configuration of the first branch flow path 42, as with the first branch flow path 42 according to the first embodiment, as the particles P1 having small particle sizes, among the plurality of particles P contained in the separation target liquid LS flowing out from the separation flow path 30, flow, these small particles P1 can be extracted from the extraction port 50 (specifically, the first extraction port 51) connected with the first branch flow path 42.

With this configuration of the second branch flow path 43, as with the second branch flow path 43 according to the first embodiment, as the particles P2 having large particle sizes, among the plurality of particles P contained in the separation target liquid LS flowing out from the separation flow path 30, flow, these large particles P2 can be extracted from the extraction port 50 (specifically, the second extraction port 52) connected with the second branch flow path 43.

(Configuration - Extraction port)

Next, a configuration of the extraction port 50 will be described.

The extraction port 50 has substantially the same configuration as the extraction port 50 according to the first embodiment. As illustrated in FIG. 6, two extraction ports are provided in the upper surface of the container 10, specifically, in a right side portion of the upper surface of the container 10.

Specifically, of the two extraction ports 50, the first extraction port 51 is connected with the downstream end portion of the first branch flow path 42 via the second connection flow path 62, and of the two extraction ports 50, the second extraction port 52 is connected with the downstream end portion of the second branch flow path 43 via the third connection flow path 63.

With the separation device 100 described above, as with the separation device 1 according to the first embodiment, a simpler and faster separation process than the above-described conventional techniques can be achieved, thereby improving the efficiency of the separation process. Furthermore, compared with the above-described conventional techniques, damage to separated particles P that occurs during the separation process can be suppressed, whereby the usability of the separated particles P can be more reliably guaranteed.

Because the first separation flow path 31 and the third separation flow path 33 are arranged substantially horizontally alongside each other, the length of the separation device 100 in the up-down direction can be reduced compared with the separation device 1 according to the first embodiment, and the separation device 100 can be installed even in a relatively low installation space.

(Separation method)

Next, a separation method using the above-mentioned separation device 100 will be described.

The separation method is substantially the same as the separation method described in the first embodiment, and thus the description thereon will be omitted.

(Effects of Second Embodiment)

According to the second embodiment, the first separation flow path 31 is configured to have a substantially helical shape to make the plurality of particles P of the sample contained in the separation target liquid LS entirely located at and/or near an inner wall portion 30a in the first separation flow path 31 as the separation target liquid LS introduced through the inlet port 20 flows through the first separation flow path 31, the second separation flow path 32 is configured to make the plurality of particles P of the sample contained in the separation target liquid LS entirely located at and/or near an outer wall portion 30b in the third separation flow path 33 as the separation target liquid LS flowing out from the first separation flow path 31 flows through the second separation flow path 32 and into the third separation flow path 33, and the third separation flow path 33 is configured to have a substantially helical shape to make the plurality of particles P of the sample contained in the separation target liquid LS separated according to the plurality of particle sizes as the separation target liquid LS flowing out from the second separation flow path 32 flows through the third separation flow path 33, and make the plurality of particles P separated located alongside each other in an inner-outer direction from an inner wall portion 30a to the outer wall portion 30b in the third separation flow path 33. Thus, as with the separation device 1 according to the first embodiment, a simpler and faster separation process than the above conventional techniques can be achieved, thereby improving the efficiency of the separation process. Compared with the above-described conventional techniques, even if the amount of the separation target liquid LS is excessive, an increase in the labor required for the separation process can be suppressed, thereby reducing the labor required for the separation process. Furthermore, compared with the above-described conventional techniques, damage to separated particles P that occurs during the separation process can be suppressed, whereby the usability of the separated particles P is more likely to be guaranteed.

[III] Modification Examples of Embodiments

While the embodiments of the present invention are described above, the specific configurations and units of the present invention can be modified and improved as desired within the scope of the technical ideas of each invention described in the claims. Such modifications are described below.

(Regarding Technical Problems or Effects of Invention)

First, the problems to be solved and the effects of the invention are not limited to those described in the preceding paragraphs. The present invention may solve problems or achieve effects not described in the preceding paragraphs, or may solve only some of the problems or achieve only some of the effects described.

(Regarding Shape, Numerical Value, Structure, and Time Series)

Regarding the components illustrated in the embodiments and the drawings, the shapes, values, and the plurality of component structures, or interrelationships in time series may be modified and improved as desired within the scope of the technical concept of the present invention.

(Regarding Separation Target Liquid)

In the above-described first embodiment, the separation target liquid LS has been described as a liquid containing only the sample. However, this should not be construed in a limiting sense, and for example, it may be a liquid containing both the sample and a sheath liquid (the same applies to the separation target liquid LS in the second embodiment).

In this case, for example, the cross-sectional size of the first separation flow path 31 may be set to be substantially the same as the cross-sectional size of the third separation flow path 33. For example, the length of the first separation flow path 31 in the inner-outer direction at the cross section may be set to be substantially the same as the length of the third separation flow path 33 in the inner-outer direction at the cross section, and the length of the first separation flow path 31 in the orthogonal direction at the cross section may be set to be substantially the same as the length of the third separation flow path 33 in the orthogonal direction at the cross section.

The first containing portion 11 may also be provided with the inlet port 20 for introducing a sample into the separation flow path 30 and the inlet port 20 for introducing a sheath liquid into the separation flow path 30.

(Regarding separation device)

In the above-described first embodiment and the above-described second embodiment, the separation device is described as including the container 10. However, this should not be construed in a limiting sense, and for example, if the separation flow path 30 and the extraction flow path 40 are configured as tubular flow paths made of, for example, resin or glass, the container 10 may be omitted.

In the above-described first embodiment and the above-described second embodiment, the separation device is described as including the first connection flow path 61, the second connection flow path 62, and the third connection flow path 63. However, this should not be construed in a limiting sense, and for example, at least one of the first connection flow path 61, the second connection flow path 62, and the third connection flow path 63 may be omitted.

In this case, the inlet port 20 or the extraction port 50 may be directly connected with the separation flow path 30 or the extraction flow path 40.

In the above-described first embodiment and the above-described second embodiment, the separation device is described as including the extraction flow path 40, but this should not be construed in a limiting sense, and for example, the extraction flow path 40 may be omitted.

In this case, the downstream side portion of the third separation flow path 33 may branch into multiple parts, and each branch may be connected with the respective extraction ports 50.

Furthermore, in the above-described first embodiment, the container 10, the inlet port 20, the separation flow path 30, the extraction flow path 40, and the extraction port 50 are described as being integrally formed, but this should not be construed in a limiting sense, and for example, some of the container 10, the inlet port 20, the separation flow path 30, the extraction flow path 40, and the extraction port 50 may be formed separately from the others and then they may be connected using a known connecting unit.

Furthermore, in the above-described first embodiment and the above-described second embodiment, the separation device is described as including the container 10, the inlet port 20, the separation flow path 30, the extraction flow path 40, and the extraction port 50, but this should not be construed in a limiting sense. For example, in addition to these configurations, if the first extraction port 51 and the second extraction port 52 are each provided on a side surface of the third containing portion 13, the separation device may further include a first storage unit (such as, for example, a known reservoir) that stores the particles P extracted from the first extraction port 51 and a second storage unit (such as, for example, a known reservoir) that stores the particles P extracted from the second extraction port 52.

Alternatively, a promoting unit (such as, for example, a known pump) for promoting the flow of the separation target liquid LS through the separation flow path 30 and the extraction flow path 40 may be further provided. In this case, for example, the separation device may be installed on the substantially vertical placement surface S.

(Regarding container)

In the above-described first embodiment and the above-described second embodiment, the container 10 is described as being formed of a resin material, but this should not be construed in a limiting sense, and for example, may be formed of a glass material or a metal material.

In the above-described first embodiment and the above-described second embodiment, the first containing portion 11 is described as being substantially cylindrical, but it may be rectangular, cubic, or columnar. In this case, the second containing portion 12 may be omitted.

(Regarding separation flow path)

In the above-described first embodiment and the above-described second embodiment, the first separation flow path 31 and the second separation flow path 32 are described as being substantially spiral or substantially helical. However, this should not be construed in a limiting sense, and the separation flow paths may be, for example, substantially arc.

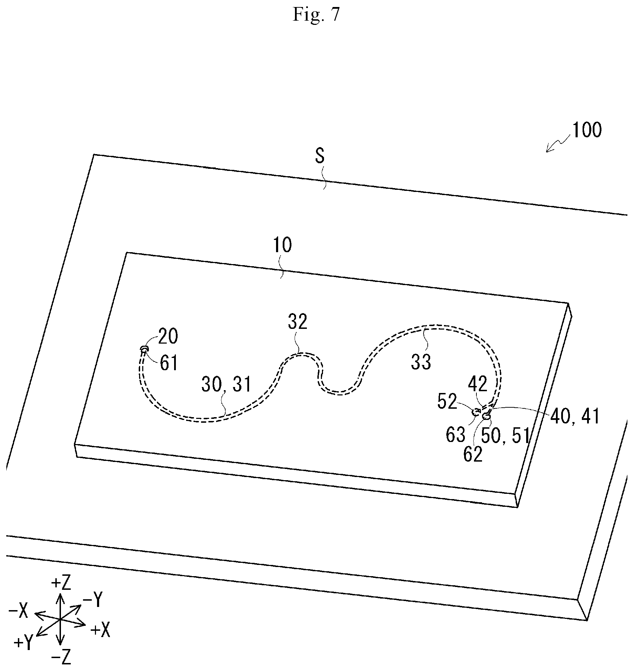

For example, as illustrated in FIG. 7, the first separation flow path 31 may be formed in a two-dimensional, substantially semicircular arc shape, and the second separation flow path 32 may be arranged horizontally alongside the first separation flow path 31 and formed in a two-dimensional, substantially semicircular arc shape that curves in the direction opposite to the first separation flow path 31. Furthermore, the downstream end portion (right end portion) of the first separation flow path 31 may be connected with the upstream end portion (left end portion) of the third separation flow path 33 via the second separation flow path 32. It is also desirable that the radii of curvature of the first separation flow path 31 and the third separation flow path 33 are set to values allowing the first separation flow path 31 and the third separation flow path 33 to function.

In addition, in the above-described first embodiment and the above-described second embodiment, the length of the third separation flow path 33 in the orthogonal direction at the cross section is set to increase from the inner wall portion 30a toward the outer wall portion 30b of the third separation flow path 33, but this should not be construed in a limiting sense, and may be set to be uniform, for example.

In the above-described first embodiment and the above-described second embodiment, the cross-sectional shape of the third separation flow path 33 is described as being substantially trapezoidal. However, this should not be construed in a limiting sense, and the shape may be, for example, substantially rectangular as illustrated in FIG. 4. Note that, while separation of the particles P is likely to be suppressed when the cross-sectional bottom surface is a curved surface (for example, circular, elliptical), such a problem should be unlikely to occur when the cross-sectional bottom surface is flat (substantially trapezoidal, substantially rectangular). In view of this, by setting the shape to be substantially rectangular, the plurality of particles P of the sample contained in the separation target liquid LS can be separated effectively according to the plurality of particle sizes in the third separation flow path 33 compared with a case where the shape is circular or elliptical. Thus, the separation performance of the third separation flow path 33 can be further improved.

In the above-described first embodiment, the third separation flow path 33 is described as being provided below the first separation flow path 31. However, this should not be construed in a limiting sense, and for example, the third separation flow path 33 may be provided above the first separation flow path 31.

In this case, the inlet port 20 may be provided near the upstream end portion (specifically, lower end portion) of the first separation flow path 31, the downstream end portion (specifically, upper end portion) of the first separation flow path 31 may be connected with the upstream end portion (specifically, lower end portion) of the third separation flow path 33 via the second separation flow path 32, and the extraction flow path 40 may be provided near the downstream end portion (specifically, upper end portion) of the third separation flow path 33. The inlet port 20 may also be provided with the above-described promotion unit.

Furthermore, in the above-described first embodiment, the third separation flow path 33 is described as being provided more on the downstream side than the first separation flow path 31, but this should not be construed in a limiting sense. For example, the third separation flow path 33 may be arranged horizontally alongside the first separation flow path 31, and the downstream end portion (lower end portion) of the first separation flow path 31 may be connected with the upstream end portion (upper end portion) of the third separation flow path 33 via the second separation flow path 32. In this case, the inlet port 20 may be provided with the above-described promotion unit.

Alternatively, the substantially spiral third separation flow path 33 wound in the same direction as the first separation flow path 31 may be arranged horizontally alongside the first separation flow path 31, and the downstream end portion (lower end portion) of the first separation flow path 31 may be connected with (the lower end portion) of the third separation flow path 33 via the second separation flow path 32. In this case, the inlet port 20 may be provided with the above-described promoting unit.

In the above-described second embodiment, the first separation flow path 31 and the third separation flow path 33 are described as being arranged horizontally alongside each other, but this should not be construed in a limiting sense. For example, the third separation flow path 33 may be arranged below the first separation flow path 31, and the downstream end portion (outer end portion) of the first separation flow path 31 may be connected with the upstream end portion (outer end portion) of the third separation flow path 33 via the second separation flow path 32.

Alternatively, the third separation flow path 33 having a helical shape wound in the same direction as the first separation flow path 31 may be provided below the first separation flow path 31, and the downstream end portion (outer end portion) of the first separation flow path 31 may be connected with the upstream end portion (inner end portion) of the third separation flow path 33 via the second separation flow path 32.

(Regarding extraction ports)

In the above-described first embodiment and the above-described second embodiment, two extraction ports 50 are provided. However, this should not be construed in a limiting sense, and for example, if only small particles P1 or large particles P2 are to be extracted among the plurality of particles P separated by the separation flow path 30, only one extraction port 50 may be provided.

In this case, one of the first branch flow path 42 and the second branch flow path 43 may be connected with the extraction port 50, and the other of the first branch flow path 42 and the second branch flow path 43 may be connected with a waste port connected with a waste storage unit (such as, for example, a known tank) for disposing of the remaining particles P of the above-described plurality of particles P separated.

For example, when extracting particles P corresponding to three or more particle sizes among the plurality of particles P separated by the separation flow path 30, the number of the extraction ports 50 provided may be three or more.

In this case, the number of branch flow paths may be three or more.

(Notes)

A separation device of note 1 is a separation device configured to separate a plurality of particles forming a sample contained in a separation target liquid, the separation device comprising: an inlet port through which the separation target liquid is introduced; a separation flow path in which the separation target liquid introduced through the inlet port flows, the separation flow path being configured to separate the plurality of particles of the sample contained in the separation target liquid according to particle sizes; and at least one extraction port configured to extract a particle of a particular particle size among the plurality of particles separated by the separation flow path, wherein the separation flow path includes: a first separation flow path connected with the inlet port; a second separation flow path connected with the first separation flow path; and a third separation flow path connected with the second separation flow path, the first separation flow path is configured to have a substantially arc shape, a substantially spiral shape, or a substantially helical shape, to make the plurality of particles of the sample contained in the separation target liquid entirely located at and/or near an inner wall portion in the first separation flow path as the separation target liquid introduced through the inlet port flows through the first separation flow path, the second separation flow path is configured to make the plurality of particles of the sample contained in the separation target liquid entirely located at and/or near an outer wall portion in the third separation flow path as the separation target liquid flowing out from the first separation flow path flows through the second separation flow path and into the third separation flow path, and the third separation flow path is configured to have a substantially arc shape, a substantially spiral shape, or a substantially helical shape, to make the plurality of particles of the sample contained in the separation target liquid separated according to a plurality of particle sizes as the separation target liquid flowing out from the second separation flow path flows through the third separation flow path, and make the plurality of particles separated located alongside each other in an inner-outer direction from an inner wall portion to the outer wall portion in the third separation flow path.

The separation device of note 2 according to the separation device of note 1, wherein the second separation flow path is formed in a substantially S shape.

The separation device of note 3 according to the separation device of note 1 or 2, wherein a length of the third separation flow path in the inner-outer direction at a cross section is set to be same as a length of the first separation flow path in the inner-outer direction at a cross section, and a length of the third separation flow path in an orthogonal direction orthogonal to the inner-outer direction at the cross section is set to be longer than a length of the first separation flow path in the orthogonal direction at the cross section.

The separation device of note 4 according to the separation device of note 3, wherein the length of the third separation flow path in the orthogonal direction at the cross section is set to increase toward the outer wall portion from the inner wall portion of the third separation flow path.

The separation device of note 5 according to the separation device of note 3, wherein the cross section of the third separation flow path is set to have a substantially rectangular shape.

The separation device of note 6 according to the separation device of note 1 or 2, wherein the first separation flow path is formed in a substantially spiral shape with multiple turns in a vertical direction, the third separation flow path is formed in a substantially spiral shape with multiple turns in the vertical direction and in a direction opposite to the first separation flow path, the third separation flow path is located more on a downstream side than the first separation flow path, and a downstream end portion of the first separation flow path and an upstream end portion of the third separation flow path are connected via the second separation flow path.

The separation device of note 7 according to the separation device of note 6, wherein the first separation flow path has a uniform radius of curvature, and/or the third separation flow path has a uniform radius of curvature.

The separation device of note 8 according to the separation device of note 1 or 2 further comprising an extraction flow path connected with the third separation flow path and a plurality of the extraction ports, wherein the extraction flow path includes: a main flow path in which the separation target liquid flowing out from the third separation flow path and containing the plurality of particles separated according to the plurality of particle sizes by the separation flow path flows; and a plurality of branch flow paths in which particles of different particle sizes, among the plurality of particles contained in the separation target liquid flowing out from the main flow path, respectively flow, the branch flow paths branching off from the main flow path, each of the branch flow paths being connected with any of the plurality of extraction ports.

The separation device of note 9 according to the separation device of note 1 or 2, wherein the sample is a biological sample containing a plurality of cells.

A separation method of separating of note 10 is a separation method of separating a plurality of particles forming a sample contained in a separation target liquid using a separation device including an inlet port, a separation flow path connected with the inlet port, and an extraction port connected with the separation flow path, the separation flow path including a first separation flow path that is connected with the inlet port and has a substantially arc shape, a substantially spiral shape, or a substantially helical shape, a second separation flow path connected with the first separation flow path, and a third separation flow path that is connected with the second separation flow path and has a substantially arc shape, a substantially spiral shape, or a substantially helical shape, the separation method comprising: an introduction step of introducing the separation target liquid into the separation flow path through the inlet port; a separation step of separating the plurality of particles of the sample contained in the separation target liquid according to particle sizes as the separation target liquid, introduced in the introduction step, flows through the separation flow path; and an extraction step of extracting a particle of a particular particle size, among the plurality of particles separated in the separation step, through the extraction port, wherein the separation step includes a first separation step of making the plurality of particles of the sample contained in the separation target liquid entirely located at and/or near an inner wall portion in the first separation flow path as the separation target liquid, introduced in the introduction step, flows through the first separation flow path, after the first separation step, a second separation step of making the plurality of particles of the sample contained in the separation target liquid entirely located at and/or near an outer wall portion in the third separation flow path as the separation target liquid flowing out from the first separation flow path flows through the second separation flow path and into the third separation flow path, and after the second separation step, a third separation step of making the plurality of particles of the sample contained in the separation target liquid separated according to the plurality of particle sizes as the separation target liquid flowing out from the second separation flow path flows through the third separation flow path, and making the plurality of particles separated located alongside each other in an inner-outer direction from an inner wall portion to the outer wall portion in the third separation flow path.

(Effect of notes)

In the separation device according to note 1 or the separation method according to note 10, the first separation flow path is configured to have a substantially arc shape, a substantially spiral shape, or a substantially helical shape, to make the plurality of particles of the sample contained in the separation target liquid entirely located at and/or near an inner wall portion in the first separation flow path as the separation target liquid introduced through the inlet port flows through the first separation flow path, the second separation flow path is configured to make the plurality of particles of the sample contained in the separation target liquid entirely located at and/or near an outer wall portion in the third separation flow path as the separation target liquid flowing out from the first separation flow path flows through the second separation flow path and into the third separation flow path, and the third separation flow path is configured to have a substantially arc shape, a substantially spiral shape, or a substantially helical shape, to make the plurality of particles of the sample contained in the separation target liquid separated according to the plurality of particle sizes as the separation target liquid flowing out from the second separation flow path flows through the third separation flow path, and make the plurality of particles separated located alongside each other in an inner-outer direction from an inner wall portion to the outer wall portion in the third separation flow path. Thus, a simpler and faster separation process than conventional techniques (techniques that separate specific cells using a centrifuge) can be achieved, thereby improving the efficiency of the separation process. Compared with the above-described conventional techniques, even if the amount of the separation target liquid is excessive, an increase in the labor required for the separation process can be suppressed, thereby reducing the labor required for the separation process. Furthermore, compared with the above-described conventional techniques, damage to separated particles that occurs during the separation process can be suppressed, whereby the usability of the separated particles is more likely to be guaranteed.

In the separation device according to note 2, the second separation flow path is formed in a substantially S shape, so that the plurality of particles of the sample contained in the separation target liquid can be entirely located at and/or near the outer wall portion in the third separation flow path effectively compared with other shapes, whereby the function of the second separation flow path can be improved.

In the separation device according to note 3, a length of the third separation flow path in the inner-outer direction at a cross section is set to be substantially the same as a length of the first separation flow path in the inner-outer direction at a cross section, and a length of the third separation flow path in an orthogonal direction orthogonal to the inner-outer direction at the cross section is set to be longer than a length of the first separation flow path in the orthogonal direction at the cross section. Thus, the plurality of particles of the sample contained in the separation target liquid can be separated according to the plurality of particle sizes in the third separation flow path effectively comparted with other lengths, whereby the separation performance of the third separation flow path can be improved.

In the separation device according to note 4, the length of the third separation flow path in the orthogonal direction at the cross section is set to increase toward the outer wall portion from the inner wall portion of the third separation flow path. Thus, the plurality of particles of the sample contained in the separation target liquid can be separated according to the plurality of particle sizes in the third separation flow path effectively compared with a case where the third separation flow path has a uniform length in the orthogonal direction at the cross section, whereby the separation performance of the third separation flow path can be further improved.

In the separation device according to note 5, the cross section of the third separation flow path is set to have a substantially rectangular shape. Thus, the plurality of particles of the sample contained in the separation target liquid can be separated according to the plurality of particle sizes in the third separation flow path effectively compared with a case where the shape is circular or elliptical, whereby the separation performance of the third separation flow path can be further improved.

In the separation device according to note 6, the first separation flow path is formed in a substantially spiral shape with multiple turns in a vertical direction, the third separation flow path is formed in a substantially spiral shape with multiple turns in the vertical direction and in a direction opposite to that of the first separation flow path, and the third separation flow path is located more on a downstream side than the first separation flow path, and a downstream end portion of the first separation flow path and an upstream end portion of the third separation flow path are connected via the second separation flow path. Thus, compared with a case where the first separation flow path and the third separation flow path are each formed in a helical shape, the first separation flow path and the third separation flow path can function effectively, whereby the separation performance of the separation flow path can be improved. Furthermore, combination and installation of the separation device and other devices (such as, for example, devices that perform pre-processing or detection processing) and/or members (such as, for example, fixing members) can be facilitated, whereby installability of the separation device can be improved.

In the separation device according to note 7, the first separation flow path has a uniform radius of curvature, and/or the third separation flow path has a uniform radius of curvature. Thus, the distance that the separation target liquid flows through the first separation flow path and/or the third separation flow path can be made long compared with a case where the radius of curvature of the first separation flow path and/or the third separation flow path varies. This allows the first separation flow path and/or the third separation flow path to function effectively (specifically, the plurality of particles contained in the separation target liquid can be stably located at and/or near the inner wall portion in the first separation flow path when the first separation flow path has a uniform radius of curvature, and the above-described plurality of particles can be stably located alongside each other in the inner-outer direction in the third separation flow path when the third separation flow path has a uniform radius of curvature), whereby the separation performance of the separation flow path can be further improved.

In the separation device according to note 8, the extraction flow path includes: a main flow path; and a plurality of branch flow paths in which particles of different particle sizes, among the plurality of particles contained in the separation target liquid flowing out from the main flow path, respectively flow, the branch flow paths branching off from the main flow path, each of the branch flow paths being connected with any of the plurality of extraction ports. Thus, particles of different particle sizes can be respectively extracted through the plurality of extraction port, whereby the plurality of particles of different particle sizes can be efficiently extracted.

In the separation device according to note 9, the sample is a biological sample containing a plurality of cells. Thus, a simpler and faster separation process on the plurality of cells can be achieved, thereby further improving the efficiency of the separation process. Furthermore, damage to separated cells that occurs during the separation process can be suppressed, whereby the usability of the separated cells is more likely to be guaranteed.

Reference Signs List

1: separation device
10: container
11: first containing portion
12: second containing portion
12a: through hole
13: third containing portion
20: inlet port
30: separation flow path
30a: inner wall portion
30b: outer wall portion
31: first separation flow path
32: second separation flow path
33: third separation flow path
40: extraction flow path
41: main flow path
42: first branch flow path
43: second branch flow path
50: extraction port
51: first extraction port
52: second extraction port
61: first connection flow path
62: second connection flow path
63: third connection flow path
100: separation device
$D_V$: Dean vortex
$F_D$: Dean power
$F_L$: lift force
LS: separation target liquid
P: particle
P1: particle having small particle size
P2: particle having large particle size
S: placement surface

**Claims**

1. A separation device configured to separate a plurality of particles forming a sample contained in a separation target liquid, the separation device comprising:

an inlet port through which the separation target liquid is introduced;

a separation flow path in which the separation target liquid introduced through the inlet port flows, the separation flow path being configured to separate the plurality of particles of the sample contained in the separation target liquid according to particle sizes; and

at least one extraction port configured to extract a particle of a particular particle size among the plurality of particles separated by the separation flow path, wherein

the separation flow path includes:

a first separation flow path connected with the inlet port;

a second separation flow path connected with the first separation flow path; and

a third separation flow path connected with the second separation flow path,

the first separation flow path is configured to have a substantially arc shape, a substantially spiral shape, or a substantially helical shape, to make the plurality of particles of the sample contained in the separation target liquid entirely located at and/or near an inner wall portion in the first separation flow path as the separation target liquid introduced through the inlet port flows through the first separation flow path,

the second separation flow path is configured to make the plurality of particles of the sample contained in the separation target liquid entirely located at and/or near an outer wall portion in the third separation flow path as the separation target liquid flowing out from the first separation flow path flows through the second separation flow path and into the third separation flow path, and

the third separation flow path is configured to have a substantially arc shape, a substantially spiral shape, or a substantially helical shape, to make the plurality of particles of the sample contained in the separation target liquid separated according to a plurality of particle sizes as the separation target liquid flowing out from the second separation flow path flows through the third separation flow path, and make the plurality of particles separated located alongside each other in an inner-outer direction from an inner wall portion to the outer wall portion in the third separation flow path.

2. The separation device according to claim 1, wherein the second separation flow path is formed in a substantially S shape.

3. The separation device according to claim 1 or 2, wherein

a length of the third separation flow path in the inner-outer direction at a cross section is set to be same as a length of the first separation flow path in the inner-outer direction at a cross section, and

a length of the third separation flow path in an orthogonal direction orthogonal to the inner-outer direction at the cross section is set to be longer than a length of the first separation flow path in the orthogonal direction at the cross section.

4. The separation device according to claim 3, wherein the length of the third separation flow path in the orthogonal direction at the cross section is set to increase toward the outer wall portion from the inner wall portion of the third separation flow path.

5. The separation device according to claim 3, wherein the cross section of the third separation flow path is set to have a substantially rectangular shape.

6. The separation device according to claim 1 or 2, wherein

the first separation flow path is formed in a substantially spiral shape with multiple turns in a vertical direction,

the third separation flow path is formed in a substantially spiral shape with multiple turns in the vertical direction and in a direction opposite to the first separation flow path,

the third separation flow path is located more on a downstream side than the first separation flow path, and

a downstream end portion of the first separation flow path and an upstream end portion of the third separation flow path are connected via the second separation flow path.

7. The separation device according to claim 6, wherein the first separation flow path has a uniform radius of curvature, and/or the third separation flow path has a uniform radius of curvature.

8. The separation device according to claim 1 or 2 further comprising an extraction flow path connected with the third separation flow path and a plurality of the extraction ports, wherein the extraction flow path includes:

a main flow path in which the separation target liquid flowing out from the third separation flow path and containing the plurality of particles separated according to the plurality of particle sizes by the separation flow path flows; and

a plurality of branch flow paths in which particles of different particle sizes, among the plurality of particles contained in the separation target liquid flowing out from the main flow path, respectively flow, the branch flow paths branching off from the main flow path, each of the branch flow paths being connected with any of the plurality of extraction ports.

9. The separation device according to claim 1 or 2, wherein the sample is a biological sample containing a plurality of cells.

10. A separation method of separating a plurality of particles forming a sample contained in a separation target liquid using a separation device including an inlet port, a separation flow path connected with the inlet port, and an extraction port connected with the separation flow path, the separation flow path including a first separation flow path that is connected with the inlet port and has a substantially arc shape, a substantially spiral shape, or a substantially helical shape, a second separation flow path connected with the first separation flow path, and a third separation flow path that is connected with the second separation flow path and has a substantially arc shape, a substantially spiral shape, or a substantially helical shape, the separation method comprising:

an introduction step of introducing the separation target liquid into the separation flow path through the inlet port;
a separation step of separating the plurality of particles of the sample contained in the separation target liquid according to particle sizes as the separation target liquid, introduced in the introduction step, flows through the separation flow path; and
an extraction step of extracting a particle of a particular particle size, among the plurality of particles separated in the separation step, through the extraction port, wherein
the separation step includes

a first separation step of making the plurality of particles of the sample contained in the separation target liquid entirely located at and/or near an inner wall portion in the first separation flow path as the separation target liquid, introduced in the introduction step, flows through the first separation flow path,
after the first separation step, a second separation step of making the plurality of particles of the sample contained in the separation target liquid entirely located at and/or near an outer wall portion in the third separation flow path as the separation target liquid flowing out from the first separation flow path flows through the second separation flow path and into the third separation flow path, and
after the second separation step, a third separation step of making the plurality of particles of the sample contained in the separation target liquid separated according to the plurality of particle sizes as the separation target liquid flowing out from the second separation flow path flows through the third separation flow path, and making the plurality of particles separated located alongside each other in an inner-outer direction from an inner wall portion to the outer wall portion in the third separation flow path.

Fig. 1

Fig. 2

(a)

(b)

Fig. 3

(a)

(b)

Fig. 4

Fig. 5

(a)

+Z
−X ←→ +X
−Z

31
30a
LS
30b
P, P1   P2

(b)

+Z
−X ←→ +X
−Z

31
30a
LS
30b
P, P1   P2

(c)

+Z
−X ←→ +X
−Z

33
LS
30a
30b
P, P1   P2

(d)

+Z
−X ←→ +X
−Z

33
LS
30a
30b
P, P1   P2

Fig. 6

Fig. 7

## INTERNATIONAL SEARCH REPORT

| International application No. |
|---|
| **PCT/JP2024/024791** |

| A. | CLASSIFICATION OF SUBJECT MATTER |
|---|---|

*B03B 5/00*(2006.01)i; *C12M 1/00*(2006.01)i; *C12N 5/00*(2006.01)i; *G01N 15/00*(2024.01)i; *G01N 15/01*(2024.01)i
FI:   B03B5/00 Z; G01N15/01; G01N15/00 C; C12M1/00 Z; C12N5/00

According to International Patent Classification (IPC) or to both national classification and IPC

| B. | FIELDS SEARCHED |
|---|---|

Minimum documentation searched (classification system followed by classification symbols)

B03B5/00; C12M1/00; C12N5/00; G01N15/00; G01N15/01

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | US 2020/0171488 A1 (MASSACHUSETTS INSTITUTE OF TECHNOLOGY) 04 June 2020 (2020-06-04)<br>    paragraphs [0137]-[0148], fig. 1-9B | 1-5, 8-10 |
| Y | | 6-7 |
| Y | JP 2022-037418 A (SONY GROUP CORPORATION) 09 March 2022 (2022-03-09)<br>    paragraphs [0012]-[0019], fig. 4-5, 7-8, 10 | 6-7 |
| Y | US 2015/0290369 A1 (BIOMET BIOLOGICS, LLC) 15 October 2015 (2015-10-15)<br>    paragraphs [0024]-[0056], fig. 1-10 | 6-7 |

☐ Further documents are listed in the continuation of Box C.     ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **04 September 2024** | **24 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

| | International application No. |
| --- | --- |
| | **PCT/JP2024/024791** |

| Patent document cited in search report | Publication date (day/month/year) | Patent family member(s) | Publication date (day/month/year) |
| --- | --- | --- | --- |
| US 2020/0171488 A1 | 04 June 2020 | WO 2020/102533 A1 description, page 11, line 18 to page 45, line 10, fig. 1-9B | |
| JP 2022-037418 A | 09 March 2022 | US 2024/0027424 A1 paragraphs [0072]-[0234], fig. 4-5, 7-8, 10 <br> WO 2022/044600 A1 | |
| US 2015/0290369 A1 | 15 October 2015 | WO 2015/157017 A1 paragraphs [0024]-[0056], fig. 1-10 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012149641 A